# EUROPEAN PATENT APPLICATION

(11) **EP 1 600 156 A2**
(43) Date of publication of application: **30.11.2005**
(21) Application number: 04738449.0
(22) Date of filing: 05.03.2004
(51) Int. Cl.: A61K 31/13

(54) **COMBINATION TREATMENT FOR IMPAIRED MOTOR FUNCTION IN PARKINSON'S DISEASE**

(30) Priority: 05.03.2003 US 452077 P; 05.03.2003 US 452055 P
(71) Applicant: Osmotica Corp., Road Town, Tortola (VG)
(72) Inventor: VERGEZ, Juan, A., 1643 Buenos Aires (AR); LANIER, Alan, B., Raleigh, NC 27614 (US); FELEDER, Ethel, C., 1426 Buenos Aires (AR); MEYER, Glenn, A., Wilmington, NC 28403 (US); RICCI, Marcelo, A., 1419 Buenos Aires (AR); FAOUR, Joaquina, 1426 Buenos Aires (AR)
(74) Representative: Weber, Thomas
(86) International application number: PCT/CR2004/000003
(87) International publication number: WO 2004/087116

(57) **Abstract**

The invention provides a method, and dosage form therefor, of treating impaired motor function associated with Parkinson's disease, anti-Parkinson's drug treatment, e.g. L-Dopa therapy, and/or dementia associated with Parkinson's disease. The invention includes the combined administration of an NMDA receptor antagonist and an antidepressant, e.g., the combination of amantadine and citalopram or venlafaxine, or an NMDA receptor antagonist and an anxiolytic agent, e.g., amantadine and buspirone or trazodone, for the amelioration of undesired tremors, akinesia, dyskinesia, or bradykinesia associated with one or more different disorders or diseases.

The drugs can be included in a single dosage form. One embodiment includes a combination dosage form containing each drug in controlled release forms. Another embodiment includes a combination dosage form providing a controlled release of an NMDA receptor antagonist and a rapid release of a neuroactive agent after administration to a subject.

## Description

### FIELD OF THE INVENTION

This invention pertains to a method, and dosage form therefore, of treating impaired motor function associated with Parkinson's disease, anti-Parkinson's drug treatment, and/or dementia associated with Parkinson's disease. More particularly, it pertains to the combined oral administration of an NMDA receptor antagonist and a neuroactive agent for the treatment of impaired motor function.

### BACKGROUND OF THE INVENTION

Antidepressants are widely known for use in treating depression in different disease states. Antidepressant use in the treatment of depression associated with Parkinson's disease, Alzheimer's disease and various types of dementia is well known. Many patients have reportedly been administered an antidepressant while also receiving anti-Parkinson's, anti-Alzheimer's or anti-dementia drug therapy. Various scientific publications and patents specifically mention the use of citalopram in treating depression associated with these disorders. However, citalopram is not generally known for its use in the treatment of tremors associated with these disorders.

Citalopram, an SSRI antidepressant that is selective for the 5HT1 receptor, is available commercially in immediate release tablet or solution form under the trademarks CELEXA™ and LEXAPRO™ (s-citalopram enantiomer). Citalopram has been used, with intermittent success, in combination with other drugs for treating depression in patients with Parkinson's disease. Heinonen et al. (Drug Safety, (July 1998), 19(1), 11-22) disclose the combined administration of citalopram with selegiline (deprenyl). They disclose the administration of selective serotonin reuptake inhibitors (SSRI's), such as citalopram, to Parkinson's patients undergoing treatment with selegiline. Rihmer et al. (International J. Psychiatry in Clin. Prac., (June 2000), 4(2), 123-125) disclose the results of a study evaluating the combined administration of selegiline and citalopram for the treatment of depression in patients with Parkinson's disease. Linasazoro (Parkinsonism & Related Disorders, (APR 2000), 6(2), 111-113) disclose the results of a case history wherein addition of citalopram to the anti-Parkinson's drug regime of a single patient actually worsened her motor symptoms.

Dell' Agnello et al. (Clin. Neuropharmacol., (JUL-AUG 2001), 24(4), 221-227) disclose the results of a study evaluating the effect of SSRI's on the extrapyramidal symptoms associated with Parkinson's disease. They report that SSRI's do not significantly worsen extrapyramidal symptomatology and may ameliorate depression in patients with Parkinson's disease. Other reports suggest that the use of SSRI antidepressants concomitant with anti-Parkinson drug therapy actually worsens motor impairment.

Korsgaard et al. (Clin. Neuropharmacol., (1986), 9(1), 52-7) report that citalopram has no significant effect in treating tardive dyskinesia or Parkinsonism.

Rampello et al. (Clin. Neuropharmacol., (JAN-FEB 2002), 25(1), 21-24) report that when combined with levodopa, citalopram induces an improvement of motor performance, in particular of subscores 23 and 31 of Unified Parkinson's Disease Rating Scale both in depressed and in nondepressed patients with Parkinson's disease.

Osmotic devices and other sustained/extended release devices containing citalopram are known in the art. Such devices have been disclosed by, for example, Kuhrts, in U.S. Patent Application Publication No. 2002/0098239, which discloses a sustained release device and suggests citalopram as a drug suitable for use in the device. Masters, in U.S. Patent Application Publication No. 2002/0106410, discloses a controlled release device, and suggests citalopram as a drug suitable for use in the device. Elema et al., in U.S. Patent Application Publication No. 2002/0160050, disclose a melt granulated modified release dosage form and claim citalopram as a drug used in the device. Chopra et al., in U.S. Patent Application Publication No. 2003/0003151, disclose a controlled release dosage form having a compressed core and claim citalopram as a drug used in the device.

BIOVAIL® is reportedly currently developing a controlled release dosage form containing citalopram for the treatment of depression; however, this product does not include amantadine.

Buspirone, a 5-HT1A agonist, is a recognized anxiolytic agent. Buspirone is primarily active in dopaminergic pathways. It has the properties of both a dopamine agonist and antagonist. Buspirone is commercially available under the trademark BUSPAR™ in rapid release tablet form.

Buspirone (BUS) has been evaluated for its effect on motor disorders associated with Parkinson's disease or levodopa therapy; however, results have been mixed and contradictory. Bonifati et al. (Clin. Neuropharmacol., (1994 Feb), 17(1), 73-82) report mixed results in the treatment of levodopa induced dyskinesia by administration of buspirone. Ross (Med. Hypotheses, (1987 Mar), 22(3), 321-8) suggests that the treatment of movement disorders with buspirone should be further evaluated. Hammerstad et al. (Clin. Neuropharmacol., (1986), 9(6), 556-560) report that buspirone is ineffective in the treatment of Parkinson's disease. Ludwig et al. (Clin. Neuropharmacol., (1986), 9(4), 373-8) report that at normal therapeutic (anxiolytic) levels, buspirone was no different than placebo in terms of its effect on dyskinesia. The combined use of an NMDA-RA and an anxiolytic agent, such as buspirone, for the treatment of motor disorders has not been disclosed.

Trazodone (TRZ) is indicated for the treatment of depression and is a classified as a triazolopyridine anxiolytic/anti-depressant, psychotropic agent or a serotonin antagonist reuptake inhibitor (SARI). TRZ is described in U.S. Patents No. 4,215,104 and No. 4,258,027. It is available commercially in immediate release form under the trademarks DESYREL™, BENEFICAT™, BIMARAN™, DEPRAX™, DESIREL™, MANEGAN™, MOLIPAXIN™, PRAGMAREL™, SIDERIL™, TAXAGON™, THOMBRAN™, TRAZALON™, TRAZOLAN™, TRAZONIL™ and TRITTICO™. The combined use of trazodone with selegiline in the treatment of depression associated with Parkinson's disease has been suggested. Ritter et al. (Ann. Clin. Psychiatry, (1997) Mar, 9(1), 7-13) report that adverse drug interactions occur upon administration of trazodone and selegiline in Parkinson's patients. According to Williams (Prim. Care, (1989 Jun), 16(2), 451-74), trazodone has a reduced occurrence of anticholinergic effects in Parkinson's patients, as compared to other drugs, but it is also very sedating. el-Awar et al. (Can. J. Neurol. Sci., (1987 Nov), 14(4), 629-31) report that antidepressants such as trazodone improve tardive and L-Dopa induced dyskinesia in patients. Sanson et al. (Riv. Neurol., (1986 Nov-Dec), 56(6), 358-64) report that trazodone significantly reduced tremors in a group of patients suffering from different types of tremors. Mastrosimone (J. Med., (1980), 11(5-6), 377-83) report the results of using different drug combinations in the treatment of patients with L-Dopa resistant Parkinson's disease.

Venlafaxine (VFX) is a recognized antidepressant with dual mechanisms of action. Venlafaxine is a mixed noradrenergic-serotonergic reuptake inhibitor. Its profile of pharmacologic activity is dose-related. At it lowest effective dose, venlafaxine primarily affects serotonin reuptake. Its noradrenergic effects come into play at higher concentrations; at doses of 300 mg/day or higher the dopaminergic system becomes activated as well. Venlafaxine is available commercially in immediate and extended release form under the trademark EFFEXOR™. It is indicated for the treatment of depression, depression with associated symptoms of anxiety, and generalized anxiety disorder.

U.S. Patents No. 6,441,048, No. 6,342,533, and No. 6,197,828 to Jerussi et al. disclose and claim pharmaceutical compositions containing derivatives of (+)-VFX or (-)-VFX and methods of using the same for the treatment of cerebral function disorders such as Parkinson's disease. The Jerussi et al. patent defines the term "method of treating Parkinson's disease" to mean "relief from the symptoms of Parkinson's disease which include, but are not limited to, slowly increasing disability in purposeful movement, tremors, bradykinesia, rigidity, and a disturbance of posture in humans." They also define the term "a method for treating cerebral function disorders" to mean "relief from the disease states associated with cerebral function disorders involving intellectual deficits which include but are not limited to, senile dementia, Alzheimer's type dementia, memory loss, amnesia/amnestic syndrome, disturbances of consciousness, coma, lowering of attention, speech disorders, Parkinson's disease, Lennox syndrome, autism, hyperkinetic syndrome and schizophrenia. Also within the meaning of cerebral function disorders are disorders caused by cerebrovascular diseases including, but not limited to, cerebral infarction, cerebral bleeding, cerebral arteriosclerosis, cerebral venous thrombosis, head injuries, and the like and where symptoms include disturbances of consciousness, senile dementia, coma, lowering of attention, speech disorders, and the like."

U.S. Patent No. 5,530,013 to Husbands et al. discloses and claims the use of venlafaxine for inducing enhancement of cognition, such as in patients suffering from Parkinson's disease. The Husbands et al. patent discloses that "It should also be understood that the present invention is intended to include all methods of, and reasons for, inducing cognition enhancement in a mammal by administering to the mammal an effective amount of venlafaxine or its analogues or pharmaceutically acceptable salts. For the purposes of the present invention, inducing cognition enhancement is to be understood as covering all prophylactic, therapeutic, progression inhibiting, remedial, maintenance, curative or other administrations, regimens or treatments of or with venlafaxine or its analogues or salts that yield the desired cognition enhancing effects in a mammal."

The use of venlafaxine for the treatment of depression associated with Parkinson's disease is also well known (See Allain et al. in British Medical Journal, (13 May 2000), 320/7245, 1287-1288; Schurer-Maly in Therapiewoche, (2001) 17/6 186-189; Poewe et al. in Journal of Neurology, Supplement, (2001) 248/3 (12-21); Okun et al. in Neurology, (26 Feb 2002) 58/4 SUPPL. 1 (S63-S70); Cunningham in J. Clin. Psych., (1994 Sep), 55 Suppl A, 90-7).

Osmotic devices containing venlafaxine for the treatment of depression are known in the art. Such devices have been disclosed by, for example, Faour et al. (U.S. Patent Application Publication No. 20010048943 and PCT International Publication No. WO 01/51041). U.S. Patents No. 6,274,171, No. 6,403,120, and No. 6,419,958 to Sherman et al. discloses an extended release venlafaxine hydrochloride formulation comprised of a capsule containing spheroids comprised of venlafaxine hydrochloride, microcrystalline cellulose, and, optionally, hydroxypropyl methylcellulose, wherein the spheroids are coated with a film.

U.S. Patent No. 6,284,276 to Rudnic et al. discloses an osmotic pharmaceutical delivery system comprised of a semipermeable wall that maintains its integrity during pharmaceutical delivery and that has a passage through it, and a composition within the semipermeable wall, wherein the composition is comprised of a pharmaceutical agent of limited solubility, a non-swelling agent that enhances the solubility of the pharmaceutical agent, and a non-swelling osmotic agent. The '276 Patent mentions amantadine as a drug that is suitable for use in the osmotic device.

Ananth et al. (*Int. Pharmacopsychiatry* (1974), 9(4), 218-29) suggests the combined administration of amantadine and an antidepressant in general for the treatment of treatment resistant depression.

Greenberg et al. (Am. J. Psychiatry (1985), 142(2), 273-4) reports on the case history of a Parkinson's patient treated for major depression with a combination of amantadine and phenelzine. The patient's depressive symptoms reportedly lessened.

Rogoz et al. (Neuropharmacology, (2002 Jun), 42(8), 1024-30) report on the synergistic combined administration of an antidepressant, such as venlafaxine, and a non-competitive N-methyl-D-aspartate receptor antagonist (NMDA-RA), such as amantadine, for the treatment of depression. There is no suggestion that the combination of amantadine and venlafaxine would be suitable for the treatment of motor disorders associated with Parkinson's or levodopa administration.

The administration of amantadine for the treatment of Parkinson's disease, Alzheimer's disease and some types of dementia is well known. Amantadine is available commercially in the United States in immediate release tablet form and syrup form under the trademark SYMMETREL™ from Endo Pharmaceutical Co. As noted in the Physician's Desk Reference 56^{th} Ed. 2002, depression, among other mood disorders, is a known adverse reaction to amantadine therapy. Moreover, amantadine is subject to undesirable interactions with a number of other drugs.

Lucetti et al. (Neurol. Sci., (2002 Sep.), 23 Suppl 2, S83-4) disclose the results of an open-label study on the use of amantadine in treating Huntington's disease, a disease characterized by chorea, cognitive and behavioral changes. They report that amantadine, a non-competitive NMDA receptor antagonist, has shown an antidyskinetic effect on levodopa-induced dyskinesias, which are known to have strict pathogenetic analogies with choreic hyperkinesias. They evaluated the antidyskinetic efficacy of amantadine and its effects on cognitive and behavioural symptoms. Eight HD patients received oral amantadine (100 mg tid) unblinded for a 1-year period. A significant reduction of dyskinesias was reported (p<0.01). No changes were observed in neuropsychologic and psychiatric assessments after 6 and 12 months of therapy.

Budipine (BY-701; 1-Tert-butyl-4, 4-diphenylpiperidine) is an NMDA-RA currently marketed by LUNDBECK A/S in Germany under the trademark PARKISAN™. It is available as the hydrochloride salt. The product is available in tablet form for administration about three times daily. Its coadministration with levo-Dopa reportedly increases dopamine levels in the brain. Budipine is a potent non-dopaminergic antiparkinsonian drug with pharmacological effects that are not comparable to those of conventional drugs applied in Parkinsonian pharmacotherapy. Budipine experimentally increased the brain content of noradrenaline, dopamine, serotonin, and histamine. The dopamine, serotonin, noradrenaline, gamma aminobutyric acid (GABA), and endorphine receptor affinities were not altered, but N-methyl-D-aspartate (NMDA) and sigma receptor affinities were increased as shown by in vivo and in vitro trials with budipine. MPTP (N-methyl-4-phenyl- 1,2,3,6-tetrahydropyridine) and MPP+ antagonistic effects have also been demonstrated. Budipine also shows neuroprotective as well as symptomatic antiparkinsonian effects. In two randomized, double-blind, multicenter, placebo-controlled studies, relevant therapeutic effects have been observed in previously untreated, so-called "de-novo" parkinsonian patients and in subjects in later stages of the disease. Budipine significantly reduces akinesia, rigidity, and tremor. Optimal effects of budipine can be seen 4-6 weeks after starting treatment with this substance. Budipine can reportedly be added to all available antiparkinsonian drug therapies. An open, prospective, long-term study of 2532 patients with Parkinson's disease (Study BY701/01A) confirmed the favorable safety and tolerability profiles of budipine (*J*. *Neurol.* (2000 Apr); 247 Suppl 2:II19-24). The combined administration of budipine and amantadine to a Parkinson's patient has been reported. No references to date suggest the coadministration of budipine with an antidepressant or an anxiolytic agent.

Controlled or sustained release dosage forms containing budipine have not been prepared; however, they have been suggested. U.S. Patent No. 6,638,528 to Kanios discloses a sustained release transdermal formulation. Kanios suggests the inclusion of budipine in the formulation. U.S. Patent Application Publication No. 2003/180332 discloses an injectable dosage form for the sustained release of an active agent over a period of 12 to 24 hours. Bupidine is suggested for use in the formulation. U.S. Patent No. 6562363 to Mantelle et al. discloses a bioadhesive topical formulation for the administration of active agents via a mucous membrane. Bupidine is suggested for use in the formulation. Williams et al. (U.S. Patent No. 6,638,981) discloses an oil-in-water emulsion formulation for the topical administration of an antidepressant, an NMDA-RA for the treatment of neuropathic pain. Bupidine is suggested for use in the formulation. U.S. Patent No. 6,024,976 to Miranda et al. discloses a sustained release transdermal formulation, which might include bupidine. PCT International Publication No. WO 03/011255 to Dudhara et al. discloses gastric retention-based controlled release dosage form. Bupidine is suggested for use in the dosage form.

U.S. Patent No. 6,479,553 to McCarthy suggests the use of an NMDA-RA such as amantadine, MK-801 or memantine, among others, as an antidepressant.

Controlled, sustained or extended release dosage forms containing amantadine are also known. PCT International Publication No. WO 9106291 to Ando et al. discloses a sustained release formulation containing amantadine. U.S. Patents No. 6,217,905, No. 5,221,536 and No. 5,190,763 to Ayer et al. of Alza Corporation disclose osmotic device formulations containing an anti-Parkinson's drug such as amantadine. U.S. Patent No. 6,491,949 to Faour et al. discloses an osmotic device formulation, and amantadine is listed as suitable for use in that osmotic device. The '763 Patent further discloses the combined administration of two different anti-Parkinson's drug from a single osmotic device. Citalopram nor any of the other neuroactive agents claimed herein are suggested or disclosed as a drug suitable for use in the combination dosage form.

U.S. Patent Application Publication No. 2002/0035105 to Caruso discloses a method of treating neuropathic pain by the combined administration of an antidepressant and an NMDA receptor antagonist. Citalopram is listed as a suitable antidepressant, and amantadine is listed as a suitable NMDA receptor antagonist. The drugs can be administered separately or in the same dosage form. The drugs can be administered concurrently or sequentially. There is no exemplification or specific mention of a dosage form containing citalopram and amantadine.

One of the main issues affecting the pharmacological treatment of PD is deciding when to initiate L-dopa therapy. Early administration has been shown to confer the greatest clinical benefits, although it is known that long-term treatment with L-dopa is associated with unpleasant motor side effects. Consequently, many physicians prefer to delay implementation of L-dopa as initial therapy. Also, L-dopa induces dyskinesia as a side effect.

Accordingly, the prior art does not disclose or suggest the combined use of an NMDA receptor antagonist and an antidepressant or anxiolytic agent for the treatment of motor disorders, particularly those associated with Parkinson's disease.

### SUMMARY OF THE INVENTION

A method of treating one or more motor symptoms observed in patients suffering from Parkinson's disease is disclosed. The method of the invention provides neuroprotective effects, decreased "off" time, increased "on" time, enhanced motor response in patients with L-dopa motor fluctuations, and/or improved motor and activity of daily living (ADL) scores in stable L-dopa responders. In particular, the present invention provides a method of treating L-Dopa induced dyskinesia, for example as it occurs in Parkinson's patients. The method employs a combination of an NMDA receptor antagonist and a neuroactive compound, such as an antidepressant agent or anti-anxiety (anxiolytic) agent. In particular, use of the combination of AMN with CIT, VFX, BUS or TRZ, for the treatment of motor disorders in patients, is disclosed. The method is not necessarily associated with administration of a particular oral dosage form; however, a controlled/sustained/extended release dosage form, e.g. osmotic device, capsule, and matrix tablet, is disclosed herein. In one embodiment, the osmotic device contains a core comprising the NMDA receptor antagonist and a surrounding coat comprising the neuroactive compound (agent) such that the drugs are released according to different release profiles. In an alternate embodiment, the osmotic device releases both drugs in a controlled manner such that the drugs are released according to the same or different release profiles.

The present inventors have discovered that an NMDA receptor antagonist (NMDA-RA), such as amantadine, works in cooperation with a neuroactive compound, such as an antidepressant or an anxiolytic agent, to reduce the occurrence of, reduce the frequency of, reduce the severity of or substantially eliminate undesired motor symptoms associated with the disorder and diseases discussed herein.

The present invention provides a method of improving the clinical status of patients suffering from motor movement related symptoms associated with one or more different disorders. The method of the invention comprises the steps of:
orally administering an amount of NMDA-RA to a subject suffering from one or more motor symptoms associated with Parkinson's disease, anti-Parkinson's drug treatment, dementia associated with Parkinson's disease; and
orally administering an amount of neuroactive agent to the subject;
wherein the drugs are administered for a period of time sufficient to reduce the occurrence, frequency or severity of the one or more motor symptoms or to substantially eliminate the occurrence of the one or more undesired motor symptoms.

Another aspect of the invention includes a combination dosage form comprising an NMDA-RA, such as amantadine, and an antidepressant, such as citalopram or venlafaxine, each of which is independently present in its salt or salt-free form. The invention also includes a dosage form that provides an immediate and/or rapid release of antidepressant in combination with a controlled/sustained/extended release of NMDA-RA. An alternate embodiment of the invention provides a controlled release of each drug from the same dosage form.

Another aspect of the invention includes a combination oral dosage form comprising an NMDA-RA, such as amantadine, and an anxiolytic agent, such as buspirone or trazodone, each of which is independently present in its salt or salt-free form. The invention also includes a dosage form that provides an immediate and/or rapid release of anxiolytic agent in combination with a controlled/sustained/extended release of NMDA-RA. An alternate embodiment of the invention includes provides a controlled release of each drug from the same dosage form.

The composition can be included in any solid (preferably oral) dosage form including, for example, unitary, encapsulated, coated, uncoated, bi-layered, or multi-layered dosage forms. These can be a patch, troche, lozenge, stick, tablet, capsule, caplet, osmotic device, powder, candy, food product, spheroids, implant, granule, sphere, paste, pastille, pill, prill, gelcap, suppository or group of particles. In a layered dosage form, the layers are arranged concentrically, eccentrically or in a stacked arrangement as disclosed in the PCT International Application No. PCT/CR02/00006. Specific embodiments of the invention include those wherein: 1) the drugs are found within separate compositions of the same dosage form; 2) the drugs are found within the same composition of the same dosage form; 3) both drugs are released in a controlled manner; 4) the NMDA-RA is released in a controlled manner and the neuroactive agent is released in a rapid manner; 5) initial release of amantadine is delayed with respect to initial release of citalopram; 6) less than 20% of the NMDA-RA is released within 3 hours after administration of the dosage form to a subject; 7) less than 42 % of the NMDA-RA is released within 6 hours after administration of the dosage form to a subject; 8) less than 64 % of the NMDA-RA is released within 10 hours after administration of the dosage form to a subject; 9) less than 85 % of the NMDA-RA is released within 15 hours after administration of the dosage form to a subject; 10) at least 75 % of the NMDA-RA is released within 20 hours after administration of the dosage form to a subject; 11) at least 75 % of the neuroactive agent is released within 3 hours after administration of the dosage form to a subject; 12) the NMDA-RA is amantadine, and the neuroactive agent is an antidepressant such as citalopram or venlafaxine; 13) the NMDA-RA is amantadine and the neuroactive agent is an anxiolytic agent such as buspirone or trazodone.

Suitable antidepressants are selected from the group consisting of Selective Serotonin Reuptake Inhibitors (SSRI) like fluoxetine, paroxetine, sertraline, fluvoxamine citalopram, and escitalopram, and Serotonin Norepinephrine Reuptake Inhibitors (SNRI) such as venlafaxine, milnacipran, and duloxetine.

Suitable non-competitive N-Methyl-D-Aspartate receptor antagonists are represented by amantadine and memantine and newer agents such as budipine and ifenprodil.

Suitable anxiolytic agents are selected from the group consisting of benzodiazepines class like lorazepam or oxazepam and nonbenzodiazepine class like buspirone, and trazodone.

Other specific embodiments of the invention include those wherein the NMDA-RA is combined with any SSRI or SNRI antidepressant, specifically: 1) amantadine and citalopram or 2) amantadine and venlafaxine. Other specific embodiments of the invention include those wherein the NMDA-RA and the anxiolytic agent are combined as follows: 1) amantadine and buspirone or 2) amantadine and trazodone.

Target therapeutic levels for each drug will depend on the specific drug combination employed. The target therapeutic levels for AMN are in the range of about 100 ng to about 1000 ng per ml of plasma. Target therapeutic levels for CIT are in the range of about 20 ng to about 200 ng per ml of plasma. The target therapeutic levels for BUS are in the range of about 1 ng to about 20 ng per ml of plasma. Target therapeutic levels for VFX are in the range of about 20 ng to about 200 ng per ml of plasma.

Other features, advantages and embodiments of the invention will become apparent to those skilled in the art by the following description, accompanying examples.

### BRIEF DESCRIPTION OF THE FIGURES

The following drawings are part of the present specification and are included to further demonstrate certain aspects of the invention. The invention may be better understood by reference to one or more of these drawings in combination with the detailed description of the specific embodiments presented herein.
FIG. 1 depicts an AMN in vitro dissolution profile for the osmotic device tablets described in Example 2.
FIG. 2 depicts a chart of the UPDRS subscale III scores of patients 1-4 and A-D up to visit 7.
FIG. 3 depicts a chart of the UPDRS subscale IV scores of patients 1-4 and A-D up to visit 7.

### DETAILED DESCRIPTION OF THE INVENTION

Amantadine is available commercially in salt or salt-free form from companies such as Northeast General Pharmaceutical Factory (Shenyang, China). When used herein, the term amantadine (or the abbreviation AMN) refers to all salt forms thereof as well as to the salt-free form. AMN of any suitable particle size can be used according to the invention.

Memantine (GA) is available as EBIXA (H. Lundeck A/S, Denmark), AXURA® (Merz Pharmaceuticals GmbH, Germany) and AKATINOL (Merz Pharmaceuticals GmbH, Germany and Phoenix, Argentina, under license).

Citalopram is available commercially in salt or salt-free form from companies such as Sekhsaria Chemicals Private Ltd. (Maharashtra, India). When used herein, the term citalopram (or the abbreviation CIT) refers to all salt forms thereof as well as to the salt-free form. CIT of any suitable particle size can be used according to the invention.

Venlafaxine is available commercially in salt or salt-free form from companies such as Medichem S.A. (Girona, Spain) and Uquifa Mexico S.A. DE C.V. (Morelos, Mexico). When used herein, the term venlafaxine (or the abbreviation VFX) refers to all salt forms thereof as well as to the salt-free form. VFX of any suitable particle size can be used according to the invention.

Buspirone is available commercially in salt or salt-free form from companies such as Southwest Synthetic Pharmaceutical General Factory (Chongqing City, Peoples Republic of China). When used herein, the term buspirone (or the abbreviation BUS) refers to all salt forms thereof as well as to the salt-free form. BUS of any suitable particle size can be used according to the invention.

Trazodone is available commercially in salt or salt-free form from companies such as Vera Laboratories Ltd., Andra Pradesh, India. When used herein, the term trazodone (or the abbreviation TRA) refers to all salt forms thereof as well as to the salt-free form. VFX of any suitable particle size can be used according to the invention.

These compounds are in their free base, free acid, racemic, optically pure, diastereomeric and/or pharmaceutically acceptable salt forms. All such forms are considered within the scope of the present invention.

By "immediate release" is meant a release of an active agent begins within a few seconds to no more than 15 minutes after administration or exposure to an environment of use.

By "rapid release" is meant a release of an active agent to an environment over a period of 1-59 minutes or over a period of up to three hours once release has begun and release can begin within a few (0.1-30) minutes after administration or after expiration of a delay period (lag time; 30 min up to 8, 10 or 12 or more hours) after administration.

By "controlled release" is meant a release of an active agent to an environment over a period of about three hours up to about 12 hours, 16 hours, 18 hours, 20 hours, a day, or more than a day. As used herein, the term "controlled release" refers to a dosage form that provides release of a drug over an extended period of time. The drug is generally released approximately according to a predetermined manner over an extended period of time. Specific exemplary embodiments include those wherein a substantially constant amount of drug is released over an extended period of time, or drug is released slowly over an extended period of time and successfully maintains substantially constant drug levels in the blood or target tissue for approximately a predetermined period of time.

By "sustained release" is meant a controlled release of an active agent that maintains a constant drug level in the blood or target tissue.

By "extended release" is meant a controlled release of an active agent from a dosage form to an environment that allow at least a two-fold reduction in frequent dosing compared to the drug presented in a conventional dosage form (e.g., a solution or rapid releasing conventional solid dosage forms).

By "delayed release" is meant a release of an active agent to an environment that exhibits an initial delay (lag time) in the release of drug after administration, in other words, that the release of the active agent starts at any time other than promptly after administration. The period of delay is generally about 5 minutes to 12 hours, or 30 minutes to 10 hours, or 30 minutes to 8 hours or 30 minutes to 6 hours.

By "release profile" is meant a profile provided by the release rate of an active agent to an environment as a function of time.

By "zero-order release profile" is meant a release profile provided by the release of a constant amount per unit time of an active agent to an environment. By "pseudo-zero order release profile" is meant a release profile that approximates a zero-order release profile.

By "first order release profile" is meant a release profile provided by the release of a constant percentage per unit time of an initial active agent charge to an environment. By "pseudo-first order release profile" is meant a release profile that approximates a first order release profile.

Pharmaceutically acceptable salts of the drugs listed herein are included within the scope of the invention. As used herein, "pharmaceutically acceptable salts" refer to derivatives of the disclosed compounds wherein the therapeutic compound is modified by making acid or base salts thereof. Examples of pharmaceutically acceptable salts include, but are not limited to, mineral or organic acid salts of the active agent. The pharmaceutically acceptable salts include the conventional non-toxic salts, for example, from non-toxic inorganic or organic acids. For example, such conventional non-toxic salts include those derived from inorganic acids such as hydrochloric, hydrobromic, sulfuric, sulfonic, sulfamic, phosphoric, nitric and the like; and the salts prepared from organic acids such as amino acids, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and other known to those of ordinary skill in the pharmaceutical sciences. Lists of suitable salts are found in texts such as Remington's Pharmaceutical Sciences, 18th Ed. (Alfonso R. Gennaro, ed.; Mack Publishing Company, Easton, PA, 1990); Remington: the Science and Practice of Pharmacy 19^{th} Ed. (Lippincott, Williams & Wilkins, 1995); Handbook of Pharmaceutical Excipients, 3^{rd} Ed. (Arthur H. Kibbe, ed.; Amer. Pharmaceutical Assoc., 1999); the Pharmaceutical Codex: Principles and Practice of Pharmaceutics 12^{th} Ed. (Walter Lund ed.; Pharmaceutical Press, London, 1994); The United States Pharmacopeia: The National Formulary (United States Pharmacopeial Convention); and Goodman and Gilman's: the Pharmacological Basis of Therapeutics (Louis S. Goodman and Lee E. Limbird, eds.; McGraw Hill, 1992), the disclosures of which are hereby incorporated by reference.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the terms "motor symptom" or "impaired motor function" refer to motor disorders occurring in patients suffering from Parkinson's disease. These disorders may be due to the etiology of Parkinson's disease, to administration of an anti-Parkinson's drug, and/or to dementia associated with Parkinson's disease. The motor disorders can include akinesia, bradykinesia and dyskinesia as well as other kinetic motor disorders observed in patients suffering from Parkinson's disease.

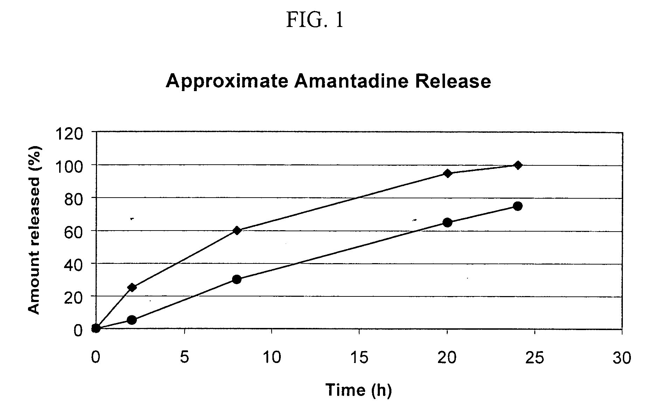

Figure 1 depicts an AMN in vitro dissolution profile for the osmotic device tablets described in Example 2. The release profile of the osmotic device of the invention will vary from that shown in Figure 1 according to the materials used to form the core and the semipermeable membrane covering the core. For example, the release profile can be influenced by the material used to form the semipermeable membrane covering the core, by the material used to form any coating on the semipermeable membrane, by the excipients present in the core, or by the presence or absence of an osmagent in the core.

Depending upon the particular combination of ingredients used to prepare the dosage form of the invention, it can be made to provide an immediate and/or rapid release of antidepressant or anxiolytic agent.

The AMN release profile for the formulation of Example 2 is generally described as follows and as depicted in FIG. 1:

| Time (h) | Maximum Percent Released | Minimum Percent Released |
|---|---|---|
| 0 | 0 | 0 |
| 2 | 25 | 5 |
| 8 | 60 | 30 |
| 20 | 95 | 65 |
| 24 | 100 | 75 |

In standard dissolution assays, the above values can vary depending upon the conditions employed. Moreover, the values may have an absolute standard deviation (STD) of ±10%, ±5% or ±3% at each given time point. For example, the amounts released at two hours might be 25±5% (maximum) and 5±5% (minimum). Accordingly, each of the values in the table above should be considered to include a standard deviation as described herein.

A controlled release dosage form can release drug according to different release profiles, for example, according to zero order pseudo-zero order, first order, pseudo-first order, sigmoidal, or pulsed release profiles. A particular release profile for the controlled release of drug can be selected for a patient according to the patient's clinical response to the dosage form by way of which the drug was administered. For example, if a patient is not responding well to a first order release profile for AMN, a dosage form with a sigmoidal, zero order or other release profile of AMN can be evaluated in the patient.

The release of each drug in a dosage form can be optimized according to the symptom and patient being treated. For example, in one patient a dual controlled release dosage form, wherein the NMDA-RA and the neuroactive agent are both released in a controlled manner; may work best; whereas in another patient, combined controlled release/rapid release dosage form, wherein the NMDA-RA is released in a controlled manner and the neuroactive agent is released in a rapid manner, may work best.

The tablets of the invention will generally provide clinically effective amounts of the NMDA-RA for a period of not less than 3 hours and not more than 30 hours, not less than 6 hours and not more than 24 hours, not less than 12 hours and not more than 24 hours, or not more than 24 hours.

The controlled release composition generally begins to release NMDA-RA within about 0-1 hours or 0-3 hours after administration or within less than about 4 hours after administration.

The term "neuroactive agent" is taken to mean a compound that exerts its pharmacologic activity in the brain of a subject. A neuroactive agent is exemplified by an antidepressant agent or anxiolytic agent.

When the antidepressant or anxiolytic agent is present in rapid release form, it can be an immediately dissolving composition that dissolves in the buccal cavity, stomach, jejunum or duodenum. The rapid release composition releases all of the antidepressant or anxiolytic agent within three hours after administration.

The oral dosage form can also provide a delay period in the release of NMDA-RA or the other drug such that, for example, the antidepressant is delivered to the stomach, jejunum, ileum or duodenum, while the NMDA-RA is released in the distal portions of the GI tract, downstream of the stomach, in a subject to which the dosage form is administered. A longer delay period can be provided such that at least one of the drugs is delivered to the small intestine and/or colon after the delay period.

The immediate release composition will release all of the neuroactive agent within 3 hours after administration to a subject (or exposure to an aqueous environment) and at least 65% of the neuroactive agent within about 60 minutes after administration (or exposure to an aqueous environment).

The rapid and/or immediate release composition is a water soluble and/or erodible composition generally comprising an inert and non-toxic material that is at least partially, and optionally substantially completely, soluble and/or erodible in an environment of use. The composition comprises synthetic or natural material that, through selective dissolution and/or erosion, provides delivery of neuroactive agent. Selection of materials suitable for the composition will depend upon the desired release rate of drug from the composition. Exemplary materials are disclosed in U.S. Patents No. 4,576,604 to Guittard et al. and No. 4,673,405 to Guittard et al., and No. 6,004,582 to Faour et al. and the text Pharmaceutical Dosage Forms: Tablets Volume I, 2^{nd} Edition. (A. Lieberman. ed. 1989, Marcel Dekker, Inc.), the relevant disclosures of which are hereby incorporated by reference. In some embodiments, the composition will be soluble in saliva, gastric juices, or acidic fluids.

Materials which are suitable for making the water soluble and/or erodible composition of the invention include, by way of example and without limitation, water soluble polysaccharide gums such as carrageenan, fucoidan, gum ghatti, tragacanth, arabinogalactan, pectin, and xanthan; water-soluble salts of polysaccharide gums such as sodium alginate, sodium tragacanthin, and sodium gum ghattate; water-soluble hydroxyalkylcellulose wherein the alkyl member is straight or branched of 1 to 7 carbons such as hydroxymethylcellulose, hydroxyethylcellulose, and hydroxypropylcellulose; synthetic water-soluble cellulose-based lamina formers such as methyl cellulose and its hydroxyalkyl methylcellulose cellulose derivatives such as a member selected from the group consisting of hydroxyethyl methylcellulose, hydroxypropyl methylcellulose, and hydroxybutyl methylcellulose; croscarmellose sodium; other cellulose polymers such as sodium carboxymethylcellulose; and other materials known to those of ordinary skill in the art. Other materials that can be used for this purpose include poly(vinylpyrrolidone), polyvinylalcohol, polyethylene oxide, a blend of gelatin and polyvinyl-pyrrolidone, gelatin, glucose, saccharides, povidone, copovidone, poly(vinylpyrrolidone)-poly(vinyl acetate) copolymer. The composition can comprise other pharmaceutical excipients that do or do not alter the way in which the composition behaves when exposed to an aqueous environment. The artisan of ordinary skill will recognize that the above-noted materials include film-forming polymers.

Other materials that can be used in the composition include hydroxypropylcellulose, microcrystalline cellulose (MCC, Avicel.TM. from FMC Corp.), poly(ethylene-vinyl acetate) (60:40) copolymer (EVAC from Aldrich Chemical Co.), 2-hydroxyethylmethacrylate (HEMA), MMA, terpolymers of HEMA:MMA:MA synthesized in the presence of N,N'-bis(methacryloyloxyethyloxycarbonylamino)-azobenzene, azopolymers, and calcium pectinate.

In some embodiments, the rapid release composition will be insoluble in the fluid of a first environment of use, such as gastric juices, acidic fluids, or polar liquids, and soluble and/or erodible in the fluid of a second environment of use, such as intestinal juices, substantially pH neutral or basic fluids, or apolar liquids. In such a case, the dosage form would provide a delayed and rapid release of drug, i.e., a rapid release of drug occurs after an initial delay period or lag time has passed. A wide variety of polymeric materials are known to possess these various solubility properties and can be included in the composition. Such other polymeric materials include, by way of example and without limitation, cellulose acetate phthalate (CAP), cellulose acetate trimelletate (CAT), poly(vinyl acetate)phthalate (PVAP), hydroxypropyl methylcellulose phthalate (HP), poly(methacrylate ethylacrylate) (1:1) copolymer (MA-EA), poly(methacrylate methylmethacrylate) (1:1) copolymer (MA-MMA), poly(methacrylate methylmethacrylate) (1:2) copolymer, Eudragit™ L-30-D (MA-EA, 1:1), Eudragit™ L-100-55 (MA-EA, 1:1), hydroxypropylmethylcellulose acetate succinate (HPMCAS), Coateric™ (PVAP), Aquateric™ (CAP), AQOAT™ (HPMCAS), poly(vinylpyrrolidone)-vinyl acetate copolymer, such as the material supplied by BASF under its Kollidon VA64 trademark, povidone, which is supplied by BASF under its Kollidon K 30 trademark, hydroxypropyl methylcellulose, which is supplied by Dow under its Methocel E-15 trademark, and combinations thereof. The composition can also comprise dissolution aids, stability modifiers, and bioabsorption enhancers.

One embodiment of the invention includes a multi-layered osmotic device comprising a core, a membrane surrounding the core and having a passageway there through, and a neuroactive agent-containing composition surrounding the membrane. The core comprises an NMDA-RA. The membrane can be a permeable, impermeable, porous or semipermeable membrane. The membrane can comprise one or more passageways and/or pores. In this embodiment, the neuroactive agent-containing composition is generally an immediate and/or rapid release composition. The osmotic device can comprise additional layers interposed or surrounding the above-described layers.

Such osmotic devices can be made according to U.S. Patent No. 4,014,334 to Theeuwes et al., U.S. Patent No. 4,576,604 to Guittard et al., Argentina Patent No. 234,493, U.S. Patent No. 4,673,405 to Guittard et al., U.S. Patent No. 5,558,879 to Chen et al., U.S. Patent No. 4,810,502 to Ayer et al., U.S. Patent No. 4,801,461 to Hamel et al., U.S. Patent No. 5,681,584 to Savastano et al., U.S. Patent No. 3,845,770, U.S. Patent No. 4,008,719 to Theeuwes et al., U.S. Patent No. 4,058,122 to Theeuwes et al., U.S. Patent No. 4,116,241 to Theeuwes et al., U.S. Patent No. 4,160,452 to Theeuwes, U.S. Patent No. 4,256,108 to Theeuwes, Argentina Patent No. 199,301, and other patent and scientific literature references concerning the preparation of osmotic devices, the entire disclosures of which are hereby incorporated by reference, and other osmotic device formulations known to those of ordinary skill in the art. Still other suitable references, the entire disclosures of which are hereby incorporated by reference, concerning the preparation of osmotic devices are U.S. Patent No. 6,004,582 to Faour et al, U.S. Patent No. 6,352,721 to Faour, U.S. Patent No. 6,491,949 to Faour et al, U.S. Patent Application Publication No. US2002/0099361 to Faour, and PCT International Application No. PCT/CR02/00006 to Vergez et al.

Osmotic devices such as those described by Faour et al. (U.S. 6,004,582) are particularly advantageous for delivering two different drugs from a single osmotic device tablet. Faour et al. disclose osmotic device formulations comprising a controlled release drug-containing core combined with a rapid release coating.

Controlled release formulations containing the pharmaceutical formulation of the invention can be made according to Biorelated Polymers and Gels: Controlled Release and Applications in Biomedical Engineering (ed. Teruo Okano; 1998); Encyclopedia of Controlled Drug Delivery (ed. Edith Mathiowitz; 1999); Future Strategies for Drug Delivery with Particulate Systems (ed. J.E. Diederichs; 1998); Controlled Release Series (ed. J.M. Anderson; 1987); Controlled Drug Delivery Series (Ed. S.D. Bruck; 1983); Controlled Release of Drugs Series (ed. M. Rosoff; 1989); Controlled Release Technology: Pharmaceutical Applications (ACS Symposium Series No. 348) (eds. P.I. Lee and W.R. Good; 1987); Extended Release Dosage Forms (ed. L. Krowczynski; 1987); Handbook of Pharmaceutical Controlled Release Technology (ed. D.L. Wise; 2000); Intelligent Materials for Controlled Release (ed. S.M. Dinh; 1999); Multicomponent Transport in Polymer Systems for Controlled Release (Polymer Science and Engineering Monograph Series) (ed. A. Polishchuk; 1997); Pharmaceutical Technology: Controlled Drug Release (ed. M. Rubenstein; 1987); Polymers for Controlled Drug Delivery (ed. P.J. Tarcha; 1991); Tailored Polymeric Materials for Controlled Delivery Systems (ACS Symposium Series No. 709) (ed. I. McCulloch; 1998); Oral Colon-Specific Drug Delivery (ed. D.R. Friend, 1992); and other publications known to those of ordinary skill in the art, the entire disclosures of which are hereby incorporated by reference

A semipermeable membrane is formed of a material that is substantially permeable to the passage of fluid from the environment of use to the core and substantially impermeable to the passage of active agent from the core. Many common materials that form a semipermeable wall which are known by those of ordinary skill in the art of pharmaceutical sciences are suitable for this purpose. Exemplary materials are cellulose esters, cellulose diesters, cellulose triesters, cellulose ethers, cellulose ester-ether, cellulose acylate, cellulose diacylate, cellulose triacylate, cellulose acetate, cellulose diacetate, cellulose triacetate, cellulose acetate propionate, cellulose acetate butyrate and ethylcellulose. cellulose esters, cellulose ethers and cellulose esters-ethers. The most preferred semipermeable membrane material is cellulose acetate, commercially available from Eastman Chemical Products. The semipermeable membrane can also contain flux enhancing agents which increase the volume of fluid imbibed into the core, such as sugar, mannitol, sucrose, sorbitol, sodium chloride, potassium chloride, polyethylene glycol (weight av. molecular weight 380-3700), propylene glycol, hydroxypropyl cellulose, hydroxypropyl methylcellulose and mixtures thereof. A preferred flux enhancer is PEG 400. The semipermeable membrane can also contain plasticizers. Suitable plasticizers for manufacturing the semipermeable membrane include sebacate, dibutylsebacate, adipate, azelate, enzoate, citrate, triethylcitrate, tributylcitrate, glyceroltributyrate, acetyltributylcitrate, acetyltriethylcitrate, stearate, isoebucate, citric acid esters, diethyloxalate, acetylated monoglyceride, oils such as olive, sesame and rape seed oil, and the like. A preferred plasticizer is triacetin. It has been found that a semipermeable membrane comprising cellulose acetate (CA) and poly(ethylene glycol) (PEG), in particular PEG 400, performs well when used in combination with the other materials required in the present osmotic device. This particular combination of CA and PEG provides a semipermeable membrane that gives the osmotic device a well controlled release profile for the active agent in the core and that retains its chemical and physical integrity in the environment of use. The ratio of CA:PEG generally ranges from about 50-99% by weight of CA: about 50-1% by weight of PEG, and about 95% by weight of CA: about 5% by weight of PEG. The ratio can be varied to alter permeability and ultimately the release profile of the osmotic device. Other suitable materials can include a selected member of the group of cellulose acylates such as cellulose acetate, cellulose diacetate, cellulose triacetate and combinations thereof. Many suitable polymers, include those disclosed in Argentine Patent No. 199,301, U.S. Patent No. 6,004,582 and references cited herein, the disclosures of which are hereby incorporated by reference.

Representative materials include a member selected from the group consisting of cellulose acylate, cellulose diacylate, cellulose triacylate, cellulose acetate, cellulose diacetate, cellulose triacetate, mono, di and tricellulose alkanylates, mono, di and tricellulose aroylates, and the like. Exemplary polymers include cellulose acetate having a D.S. up to 1 and an acetyl content up to 21%; cellulose acetate having an acetyl content of 32 to 39.8%; cellulose diacetate having a D.S. of 1 to 2 and an acetyl content of 21 to 35%; cellulose triacetate having a D.S. of 2 to 3 and an acetyl content of 35 to 44.8%; and the like. More specific cellulosic polymers include cellulose propionate having a D.S. of 1.8 and a propionyl content of 39.2 to 45% and a hydroxyl content of 2.8 to 5.4%; cellulose acetate butyrate having a D.S. of 1.8, an acetyl content of 13 to 15% and a butyryl content of 34 to 39%; cellulose acetate butyrate having an acetyl content of 2 to 29%; a butyryl content of 17 to 53% and a hydroxyl content of 0.5 to 4.7%; cellulose triacylates having a D.S. of 2.9 to 3 such as cellulose trivalerate, cellulose trilaurate, cellulose tripalmitate, cellulose trisuccinate, and cellulose trioclanoate; cellulose diacylates having a D.S. of 2.2 to 2.6 such as cellulose disuccinate, cellulose dipalmitate, cellulose dioctanoate, cellulose dipentate, and the like. Additional semipermeable polymers include acetaldehyde dimethyl acetate, cellulose acetate ethyl carbamate, cellulose acetate phthalate for use in environments having a low ph, cellulose acetate methyl carbamate, cellulose acetate dimethyl aminoacetate, semipermeable polyamides, semipermeable polyurethanes, semipermeable sulfonated polystyrenes, cross-linked selectively semipermeable polymers formed by the coprecipitation of a polyanion and a polycation as disclosed in U.S. Patents No. 3,173,876, No. 3,276,586, No. 3,541,005, No. 3,541,006, and No. 3,546,142; semipermeable polymers as disclosed by Loeb and Sourirajan in U.S. Pat. No. 3,133,132; lightly cross-linked polystyrene derivatives; cross-linked poly(sodium styrene sulfonate), cross-linked poly(vinylbenzyltrimethyl ammonium chloride), semipermeable polymers exhibiting a fluid permeability of 10.sup.-5 to 10.sup.-1 (cc.mil/cm.sup.2.hr.atm) expressed as per atmosphere of hydrostatic or osmotic pressure difference across the semipermeable wall. These and others polymers are disclosed in U.S. Patents No. 3,845,770, No. 3,916,899, No. 4,765,989 and No. 4,160,020; and in Handbook of Common Polymers (Scott, J. R. and Roff, W. J., eds.; 1971; CRC Press, Cleveland, Ohio).

The osmotic device of the invention comprises at least one passageway (pore, hole, or aperture) that communicates the exterior of the semipermeable wall with the core of the device. The passageway can be formed according to any of the known methods of forming passageways in a membrane. Such methods include, for example, 1) drilling a hole through the semipermeable membrane with a bit or laser; 2) including a water soluble material within the composition that forms the semipermeable membrane such that a pore forms when the osmotic device is in an aqueous environment of use; 3) punching a hole through the semipermeable membrane; 4) employing a tablet punch having a pin to punch a hole through the semipermeable lamina; 5) form a rupture in the membrane during use and after exposure of the osmotic device to an aqueous environment. The passageway can pass through the membrane and one or more of any other lamina coated onto the membrane or between the membrane and the core. The passageway(s) can be shaped as desired. In some embodiments, the passageway is laser drilled and is shaped as an oval, ellipse, slot, slit, cross or circle.

Methods of forming passageways in membranes of osmotic devices are disclosed in U.S. Patents No. 4,088,864 to Theeuwes et al., No. 4,016,880 to Theeuwes et al., No. 3,916,899 to Theeuwes et al., No. 4,285,987 to Ayer et al., No. 4,783,337 to Wong et al., No. 5,558,879 to Chen et al., No. 4,801,461 to Hamel et al., and No. 3,845,770 to Theeuwes et al., as well as a number of scientific literature publications the disclosures of which are hereby incorporated by reference.

The core of the osmotic device tablet of the present invention comprises an NMDA-RA, at least one pharmaceutically acceptable excipient and optionally one or more other materials. Generally, the tablet formulations will comprise about 0.1-99.9% by weight of NMDA-RA in the uncoated tablet core. Acceptable ranges may vary according to the desired therapeutic response, the tablet size, the amount and type of excipients used in the core of the device, the amount of neuroactive agent used and the intended use of the osmotic device.

When the controlled release tablet is an osmotic device, osmotically effective solutes, osmotic agents or osmagents are sometimes added. These osmagents can aid in either the suspension or dissolution of the active agent in the core. Exemplary osmagents include organic and inorganic compounds such as salts, acids, bases, chelating agents, sodium chloride, lithium chloride, magnesium chloride, magnesium sulfate, lithium sulfate, potassium chloride, sodium sulfite, calcium bicarbonate, sodium sulfate, calcium sulfate, calcium laetate, d-mannitol, urea, tartaric acid, raffinose, sucrose, alpha-d-lactose monohydrate, glucose, combinations thereof and other similar or equivalent materials which are widely known in the art. Osmagents can also be incorporated to the core of the osmotic device to control the release of active agent therefrom. U.S. Patent No. 4,077,407 to Theeuwes et al., the entire disclosure of which is hereby incorporated by reference, discloses suitable osmagents.

The osmotic device of the invention can also be made to include a displacement composition in the core. Such devices are made by including a swellable material in the displacement composition. Upon exposure to and imbibition of water by the swellable material, the displacement composition swells thereby forcing a drug-containing composition, e.g. AMN-containing composition, out of the dosage form and into the environment of use. These types of osmotic devices are well known and are disclosed in many of the references cited herein.

A coating, coat, shell, membrane, lamina or wall (these terms are used interchangeably) surrounding the core of a controlled release dosage form can be permeable, impermeable or semipermeable. The term "permeable" is taken to mean a wall that is permeabe to the passage of fluid and drug. The term "impermeable" is taken to mean a wall that does not permit passage of either fluid or drug unless a passageway or pore is formed in the wall. The term "semipermeable" is taken to mean a wall that is permeable to the passage of fluid but impermeable to the passage of drug. A dosage form of the invention can have either of these types of walls.

The tablets of the invention can also comprise adsorbents, antioxidants, buffering agents, colorants, flavorants, sweetening agents, tablet antiadherents, tablet binders, tablet and capsule diluents, tablet direct compression excipients, tablet disintegrants, tablet glidants, tablet lubricants, tablet or capsule opaquants and/or tablet polishing agents.

As used herein, the term "adsorbent" is intended to mean an agent capable of holding other molecules onto its surface by physical or chemical (chemisorption) means. Such compounds include, by way of example and without limitation, powdered and activated charcoal and other materials known to one of ordinary skill in the art.

As used herein, the term "antioxidant" is intended to mean an agent which inhibits oxidation and thus is used to prevent the deterioration of preparations by the oxidative process. Such compounds include, by way of example and without limitation, ascorbic acid, ascorbyl palmitate, butylated hydroxyanisole, butylated hydroxytoluene, hypophophorous acid, monothioglycerol, propyl gallate, sodium ascorbate, sodium bisulfite, sodium formaldehyde sulfoxylate and sodium metabisulfite and other materials known to one of ordinary skill in the art.

As used herein, the term "buffering agent" is intended to mean a compound used to resist change in pH upon dilution or addition of acid or alkali. Such compounds include, by way of example and without limitation, potassium metaphosphate, potassium phosphate, monobasic sodium acetate and sodium citrate anhydrous and dihydrate and other materials known to one of ordinary skill in the art.

As used herein, the term "sweetening agent" is intended to mean a compound used to impart sweetness to a preparation. Such compounds include, by way of example and without limitation, aspartame, dextrose, glycerin, mannitol, saccharin sodium, sorbitol and sucrose and other materials known to one of ordinary skill in the art.

As used herein, the term "tablet antiadherents" is intended to mean agents which prevent the sticking of tablet formulation ingredients to punches and dies in a tableting machine during production. Such compounds include, by way of example and without limitation, magnesium stearate, talc, calcium stearate, glyceryl behenate, PEG, hydrogenated vegetable oil, mineral oil, stearic acid and other materials known to one of ordinary skill in the art.

As used herein, the term "tablet binders" is intended to mean substances used to cause adhesion of powder particles in table granulations. Such compounds include, by way of example and without limitation, acacia, alginic acid, carboxymethylcellulose sodium, poly(vinylpyrrolidone), compressible sugar (e.g., NuTab), ethylcellulose, gelatin, liquid glucose, methylcellulose, povidone and pregelatinized starch and other materials known to one of ordinary skill in the art.

When needed, binders may also be included in the tablets. Exemplary binders include acacia, tragacanth, gelatin, starch, cellulose materials such as methyl cellulose and sodium carboxy methyl cellulose, alginic acids and salts thereof, polyethylene glycol, guar gum, polysaccharide, bentonites, sugars, invert sugars, poloxamers (PLURONIC™ F68, PLURONIC™ F127), collagen, albumin, gelatin, cellulosics in nonaqueous solvents, combinations thereof and the like. Other binders include, for example, polypropylene glycol, polyoxyethylene-polypropylene copolymer, polyethylene ester, polyethylene sorbitan ester, polyethylene oxide, combinations thereof and other materials known to one of ordinary skill in the art.

As used herein, the term "tablet and capsule diluent" or "fillers" is intended to mean inert substances used as fillers to create the desired bulk, flow properties, and compression characteristics in the preparation of tablets and capsules. Such compounds include, by way of example and without limitation, dibasic calcium phosphate, kaolin, lactose, sucrose, mannitol, microcrystalline cellulose, powdered cellulose, precipitated calcium carbonate, sorbitol, and starch and other materials known to one of ordinary skill in the art.

As used herein, the term "tablet direct compression excipient" is intended to mean a compound used in direct compression tablet formulations. Such compounds include, by way of example and without limitation, dibasic calcium phosphate (e.g., Ditab) and other materials known to one of ordinary skill in the art.

As used herein, the term "tablet glidant" is intended to mean agents used in tablet and capsule formulations to promote flowability of the granulation. Such compounds include, by way of example and without limitation, colloidal silica, cornstarch, talc, calcium silicate, magnesium silicate, colloidal silicon, silicon hydrogel and other materials known to one of ordinary skill in the art.

As used herein, the term "tablet lubricant" is intended to mean substances used in tablet formulations to reduce friction during tablet compression. Such compounds include, by way of example and without limitation, calcium stearate, magnesium stearate, mineral oil, stearic acid, and zinc stearate and other materials known to one of ordinary skill in the art.

As used herein, the term "tablet/capsule opaquant" is intended to mean a compound used to render a capsule or a tablet coating opaque. May be used alone or in combination with a colorant. Such compounds include, by way of example and without limitation, titanium dioxide, talc and other materials known to one of ordinary skill in the art.

As used herein, the term "tablet polishing agent" is intended to mean a compound used to impart an attractive sheen to coated tablets. Such compounds include, by way of example and without limitation, carnauba wax, white wax and other materials known to one of ordinary skill in the art.

As used herein, the term "tablet disintegrant" is intended to mean a compound used in solid dosage forms to promote the disruption of the solid mass into smaller particles which are more readily dispersed or dissolved. Exemplary disintegrants include, by way of example and without limitation, starches such as corn starch, potato starch, pregelatinized and modified starches thereof, sweeteners, clays, such as bentonite, microcrystalline cellulose(e.g., Avicel), carboxymethylcellulose calcium, cellulose polyacrilin potassium (e.g., Amberlite), alginates, sodium starch glycolate, gums such as agar, guar, locust bean, karaya, pectin, tragacanth; crospovidone and other materials known to one of ordinary skill in the art.

As used herein, the term "colorant" is intended to mean a compound used to impart color to solid (e.g., tablets) pharmaceutical preparations. Such compounds include, by way of example and without limitation, FD&C Red No. 3, FD&C Red No. 20, FD&C Yellow No. 6, FD&C Blue No. 2, D&C Green No. 5, D&C Orange No. 5, D&C Red No. 8, caramel, and ferric oxide, red, other F.D. & C. dyes and natural coloring agents such as grape skin extract, beet red powder, beta-carotene, annato, carmine, turmeric, paprika, and other materials known to one of ordinary skill in the art. The amount of coloring agent used will vary as desired.

As used herein, the term "flavorant" is intended to mean a compound used to impart a pleasant flavor and often odor to a pharmaceutical preparation. Exemplary flavoring agents or flavorants include synthetic flavor oils and flavoring aromatics and/or natural oils, extracts from plants, leaves, flowers, fruits and so forth and combinations thereof. These may also include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Other useful flavors include vanilla, citrus oil, including lemon, orange, grape, lime and grapefruit, and fruit essences, including apple, pear, peach, strawberry, raspberry, cherry, plum, pineapple, apricot and so forth. Flavors which have been found to be particularly useful include commercially available orange, grape, cherry and bubble gum flavors and mixtures thereof. The amount of flavoring may depend on a number of factors, including the organoleptic effect desired. Flavors will be present in any amount as desired by those of ordinary skill in the art. Particularly flavors are the grape and cherry flavors and citrus flavors such as orange.

The present tablets can also employ one or more commonly known surface active agents or cosolvents that improve wetting or disintegration of the tablet core or layers.

Plasticizers can also be included in the tablets to modify the properties and characteristics of the polymers used in the coats or core of the tablets. As used herein, the term "plasticizer" includes all compounds capable of plasticizing or softening a polymer or binder used in invention. The plasticizer should be able to lower the melting temperature or glass transition temperature (softening point temperature) of the polymer or binder. Plasticizers, such as low molecular weight PEG, generally broaden the average molecular weight of a polymer in which they are included thereby lowering its glass transition temperature or softening point. Plasticizers also generally reduce the viscosity of a polymer. It is possible the plasticizer will impart some particularly advantageous physical properties to the osmotic device of the invention.

Plasticizers useful in the invention can include, by way of example and without limitation, low molecular weight polymers, oligomers, copolymers, oils, small organic molecules, low molecular weight polyols having aliphatic hydroxyls, ester-type plasticizers, glycol ethers, poly(propylene glycol), multi-block polymers, single block polymers, low molecular weight poly(ethylene glycol), citrate ester-type plasticizers, triacetin, propylene glycol and glycerin. Such plasticizers can also include ethylene glycol, 1,2-butylene glycol, 2,3-butylene glycol, styrene glycol, diethylene glycol, triethylene glycol, tetraethylene glycol and other poly(ethylene glycol) compounds, monopropylene glycol monoisopropyl ether, propylene glycol monoethyl ether, ethylene glycol monoethyl ether, diethylene glycol monoethyl ether, sorbitol lactate, ethyl lactate, butyl lactate, ethyl glycolate, dibutylsebacate, acetyltributylcitrate, triethyl citrate, acetyl triethyl citrate, tributyl citrate and allyl glycolate. All such plasticizers are commercially available from sources such as Aldrich or Sigma Chemical Co. It is also contemplated and within the scope of the invention, that a combination of plasticizers may be used in the present formulation. The PEG based plasticizers are available commercially or can be made by a variety of methods, such as disclosed in Poly(ethylene glycol) Chemistry: Biotechnical and Biomedical Applications (J.M. Harris, Ed.; Plenum Press, NY) the disclosure of which is hereby incorporated by reference.

The tablets of the invention can also include oils, for example, fixed oils, such as peanut oil, sesame oil, cottonseed oil, corn oil and olive oil; fatty acids, such as oleic acid, stearic acid and isotearic acid; and fatty acid esters, such as ethyl oleate, isopropyl myristate, fatty acid glycerides and acetylated fatty acid glycerides. It can also be mixed with alcohols, such as ethanol, isopropanol, hexadecyl alcohol, glycerol and propylene glycol; with glycerol ketals, such as 2,2-dimethyl-1,3-dioxolane-4-methanol; with ethers, such as poly(ethyleneglycol) 450, with petroleum hydrocarbons, such as mineral oil and petrolatum; with water, or with mixtures thereof; with or without the addition of a pharmaceutically suitable surfactant, suspending agent or emulsifying agent.

Soaps and synthetic detergents may be employed as surfactants and as vehicles for detergent compositions. Suitable soaps include fatty acid alkali metal, ammonium, and triethanolamine salts. Suitable detergents include cationic detergents, for example, dimethyl dialkyl ammonium halides, alkyl pyridinium halides, and alkylamine acetates; anionic detergents, for example, alkyl, aryl and olefin sulfonates, alkyl, olefin, ether and monoglyceride sulfates, and sulfosuccinates; nonionic detergents, for example, fatty amine oxides, fatty acid alkanolamides, and poly(oxyethylene)-block-poly(oxypropylene) copolymers; and amphoteric detergents, for example, alkyl β-aminopropionates and 2-alkylimidazoline quaternary ammonium salts; and mixtures thereof.

Various other components, not otherwise listed above, can be added to the present formulation for optimization of a desired active agent release profile including, by way of example and without limitation, glycerylmonostearate, nylon, cellulose acetate butyrate, d, l-poly(lactic acid), 1,6 - hexanediamine, diethylenetriamine, starches, derivatized starches, acetylated monoglycerides, gelatin coacervates, poly (styrene - maleic acid) copolymer, glycowax, castor wax, stearyl alcohol, glycerol palmitostearate, poly(ethylene), poly(vinyl acetate), poly(vinyl chloride), 1,3 - butylene-glycoldimethacrylate, ethyleneglycol-dimethacrylate and methacrylate hydrogels.

It should be understood, that compounds used in the art of pharmaceutical formulation generally serve a variety of functions or purposes. Thus, if a compound named herein is mentioned only once or is used to define more than one term herein, its purpose or function should not be construed as being limited solely to that named purpose(s) or function(s).

The present inventors have discovered that the dosage forms of the invention provide an improved clinical benefit over either drug alone. For example, in the case where CIT alone provides an observed clinical benefit in a subject for the treatment of motor disorders, then the combination of AMN and CIT provides an even better clinical benefit in the same subject. Likewise, in the case where AMN alone provides an observed clinical benefit in a subject for the treatment of motor disorders, then the combination of AMN and CIT provides an even better clinical benefit in the same subject. In other words, the combined administration of an NMDA and a neuroactive agent provides an improved clinical benefit over either agent alone in the amelioration of motor disorders. When administered to a patient undergoing L-Dopa therapy, the NMDA-RA, e.g. amantadine, in controlled release form, with the neuroactive agent, e.g. citalopram, in rapid release form, will reduce the dyskinesias caused by the L-dopa treatment. Consequently, this combination allows the daily dosage of L-dopa to be reduced while improving the tapering of symptoms and reducing dyskinesia associated with administration of L-dopa. More generally, the combined administration of amantadine and antidepressant or anxiolytic will provide the above-described effect. The combined administration of AMN and CIT produces improvement in motor functionality of Parkinson's disease patients and also improves motor fluctuations induced by pharmacological therapy. The combination, specifically decreases the incidence of either monophasic or biphasic dyskinesias, the "on-off" and the wearing-off phenomenon. In this regard, clinical standarized measurements, the UPDRS subscale III and IV, the AIMS, and the on-off periods are improved on a weekly basis after the administration of the combination of both drugs. For motor performance, the combination will improve patient's bradykinesia, that is one of the main cardinal symptoms of the disease.

The treatment of different types motor disorders, e.g., akinesia, bradykinesia or dyskinesia, in a patient may require different dosing regimens of the drugs. The proper amounts are easily determined clinically by observation of a patient's clinical response to a particular dosing regimen.

Those of ordinary skill in the art will appreciate that the particular amounts of NMDA-RA and neuroactive agent used in the dosage form will vary according to, among other things, the desired pharmacokinetic, pharmacologic, therapeutic and/or pharmacodynamic performance in a mammal to which it is administered.

All of the dosage forms of the invention will.provide clinically effective plasma levels of NMDA-RA and neuroactive agent for at least a predetermined period of time after administration to a subject. The amount of each drug present can be dependent or independent of each other. By way of cooperation, the drugs provide an improved clinical benefit as compared to the administration of either drug alone.

The term "clinically effective amount" is taken to mean that amount of drug that is sufficient to, after a period of time, provide a desired clinical benefit in a subject to which the drug is administered. Since the drugs provide a cooperative effect, the clinically effective amount of drug present in a given dosage form can be less than or about the same as the known-therapeutically effective amount for that drug in treating the particular indication being treated. In other words, therapeutically effective or sub-therapeutically effective amounts of either one or both drugs can be included in the dosage form. The term "therapeutically effective amount" is taken to mean the amount or quantity of active agent that is sufficient to elicit the required or desired therapeutic response, or in other words, the amount that is sufficient to elicit an appreciable biological response when administered to a patient. The term "subtherapeutically effective amount" is taken to mean an amount or quantity of active agent that is less than the therapeutically effective amount and yet sufficient to elicit the required or desired therapeutic response when administered to a patient, due to for example, a cooperative effect between drugs.

It should be noted that there is a difference between a therapeutic effect and a clinical effect. A therapeutic effect is taken to mean a beneficial effect toward reducing the occurrence of, reducing the frequency of, reducing the severity of or substantially eliminating undesired motor symptoms associated with. the effects of Parkinsonism and L-dopa induced dyskinesias discussed herein. A clinical effect can include a therapeutic effect and can also include other effects such as reduced occurrence and/or severity of toxicity, and improved tolerability of the drugs. It is expected that the combination will reduce the occurrence and/or severity of bradykinesia in a superior way than an NMDA-RA alone or a neuroactive agent alone.

The oral dosage form of the invention can assume any shape or form known in the art of pharmaceutical sciences. The device of the invention can be a pill, sphere, tablet, bar, plate, paraboloid of revolution, ellipsoid of revolution or the like. The dosage form can also include surface markings, cuttings, grooves, letters and/or numerals for the purposes of decoration, identification and/or other purposes.

A composition comprising neuroactive agent can be applied onto another layer by compression or spray coating. The sprayed coating is thinner and lighter than the compression coating, and an osmotic device including the sprayed on coating is, therefore, smaller than a similar osmotic device having a compression coat. Moreover, the use of a sprayed-on drug-containing water soluble and/or erodible coating permits the loading of higher amounts of drug than the use of a compression-coated drug-containing water soluble and/or erodible coating. A smaller size osmotic device generally results in increased patient compliance in taking the osmotic device and is therefore advantageous.

The solid dosage forms of the invention can be coated with a finish coat as is commonly done in the art to provide the desired shine, color, taste or other aesthetic characteristics. Materials suitable for preparing the finish coat are well known in the art and found in the disclosures of many of the references cited and incorporated by reference herein.

The treatment of undesired motor symptoms is accomplished by administering an NDMA-RA in combination with a neuroactive agent so that each drug is present in an amount sufficient to exert its respective desired clinical effect(s). The weight ratio of NMDA-RA to neuroactive agent can range from 1.5 to 120.

The total amount of drug present in each dosage form will be limited by the physical size of the dosage form as well as by the total amount of excipients required to be present in the dosage form in order to provide the desired clinical/therapeutic effect. The daily dose ranges for AMN, Ifenprodil, CIT, VFX, MEM, BUS, and TRA are about 100-400 mg, 5-40 mg, 20-60 mg, 37.5-225 mg, 5-40 mg, 10-60 mg and 150-600 mg, respectively. The strength of the drugs in the dosage forms of the invention are indicated as free base, free acid, or salt.

In a study being conducted (double-blind, randomized, placebo-controlled, parallel-group design, with 2 treatment phases: 3 weeks open label, up -titration AMN lead-in phase, followed by a 6 weeks add-on CIT phase, performed in Parkinson Disease patients suffering from severe bradykinesia and motor fluctuations -dyskinesias- induced by L-Dopa therapy, as described in Method B of Example 9), the Unified Parkinson Disease Rating Scale subscale III (UPDRS III), Abnormal Involuntary Movement Scale (AIMS), item 23 and 31 of the Unified Parkinson Disease Rating Scale subscale III (UPDRS III 23 and UPDRS III 31 respectively), are being used to evaluate improvement in motor performance while Unified Parkinson Disease Rating Scale subscale IV (UPDRS IV) and -AIMS are being used for motor fluctuations evaluations. The first 4 patients treated with the combination of AMN and CIT (patients 1-4), and the first 4 control patients treated with AMN and placebo (patients A-D), showed the following results during the first 6 weeks (7 visits) of treatment:

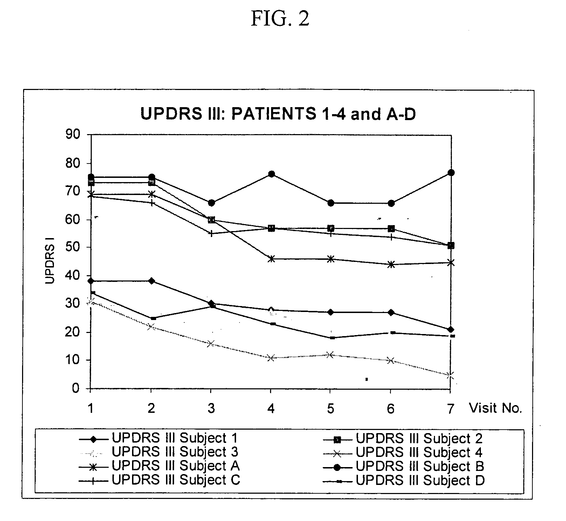

The longitudinal course of Parkinson's Disease in patients 1, 2, and 4, followed by the UPDRS III scores evaluated at the first four visits, shows an improvement in motor performance over the three weeks of treatment with amantadine only. The longitudinal course of Parkinson's Disease in patients 1, 2, and 4, followed by the UPDRS III scores evaluated at visits 5, 6, and 7, shows a greater improvement in motor performance than the improvement obtained with amantadine along. In other words, the combination of AMN and CIT shows a superior effect (Fig 2, UPDRS III patients 1, 2 and 4) over control. Placebo-treated patients showed no superior effect with the combination treatment (Fig 2, UPDRS III patients A, B, C, and D).

The longitudinal course of Parkinson's Disease in patient 3 followed by the UPDRS III scores up to visit 7 showed no improvement in motor performance with the combination treatment (Fig 2, UPDRS III patient 3).

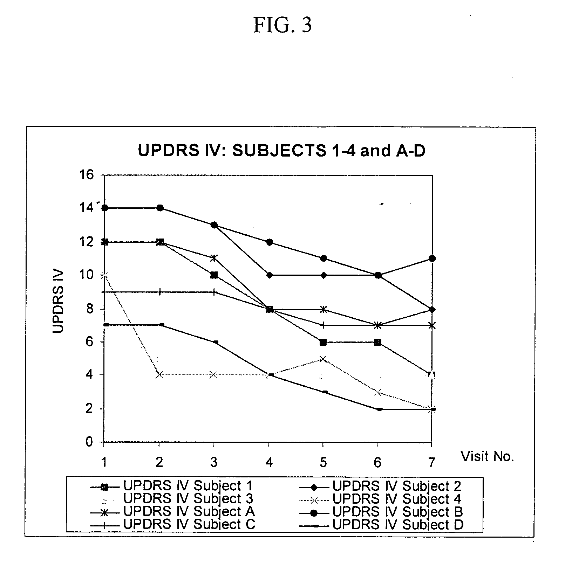

The course of the severity and characteristics of L-Dopa induced dyskinesias evaluated in patients 1, 2, and 4, by the UPDRS IV scores at the first four visits, shows an improvement in motor fluctuations over the three weeks of treatment with amantadine only. The course of the severity and characteristics of L-Dopa induced dyskinesias evaluated in patients 1, 2, and 4, followed by the UPDRS IV scores at visits 5, 6, and 7 shows a greater improvement in motor fluctuations with the combination than the improvement obtained with amantadine alone (Fig 3, UPDRS IV patients 1, 2 and 4). The course of the severity and characteristics of L-Dopa induced dyskinesias evaluated in patient 3 followed by the UPDRS IV scores up to visit 7 shows no improvement in motor fluctuations with the combination treatment (Fig 3, UPDRS IV patient 3).

The AIMS scores for treated patients showed a tendency to superior clinical benefit after treatment with AMN and CIT compared to AMN and placebo treated patients, suggesting that the combination treatment is effective in reducing dyskinesias in parkinsonian patients.

The method of orally administering an NMDA-RA, such as AMN, and a neuroactive agent, such as CIT, to a subject suffering from undesired motor movement symptoms associated with one or more disorders, such Parkinson's disease, reduces the occurrence, frequency or severity of the one or more motor movement symptoms or substantially eliminates the occurrence of the one or more motor movement symptoms.

The method of the invention provides for once or twice daily oral administration of an oral dosage form to a subject suffering from undesired motor movement symptoms associated with Parkinson's disease or dementia, the dosage form comprising a therapeutically effective or sub-therapeutically effective amount of an NMDA-RA, a therapeutically effective or sub-therapeutically effective amount of a neuroactive agent, and one or more pharmaceutically acceptable excipients, wherein when the dosage form is administered to the subject the NMDA-RA is released according to a rapid, controlled or sustained release profile, the neuroactive agent is released according to a rapid, controlled or sustained release profile, and release of one or both the NMDA-RA and the neuroactive agent is optionally delayed a lag period, whereby the occurrence, frequency or severity of the one or more motor movement symptoms is reduced or substantially eliminated.

The invention also provides for use of a combination of an amount of NMDA-RA and an amount of neuroactive agent for the manufacture of an oral medicament for the treatment of or improvement of the clinical status of a patient suffering from undesired motor movement symptoms associated with one or more disorders, such as Parkinson's disease or dementia. The medicament can be manufactured according to the methods described herein. The medicament can be used in practice of the method of treatment described herein.

The following examples should not be considered exhaustive, but merely illustrative of only a few of the many embodiments contemplated by the present invention. The methods described herein can be followed to prepare osmotic devices, tablets, and capsules according to the invention.

### EXAMPLE 1

### Multi-layered osmotic device containing amantadine

The following general method was used to prepare osmotic device that can subsequently be coated with a citalopram-containing composition according to the invention. The above method was used to prepare tablets having the following formulation:

| CORE | |
|---|---|
| Amantadine HCl | 300.0 mg |
| Osmagent | 0.10-40.00 mg |
| Diluent | 0.1-20.00 mg |
| Binder | 1.00-10.00 mg |
| Plasticizer | 0.10-6.00 mg |
| Glidant | 0.1-1.00 mg |
| Lubricant | 0.10-3.00 mg |

| COATING A (semipermeable membrane) | |
|---|---|
| Cellulose Ester | 9.50-88.50 mg |
| Plasticizer | 0.5-1.50 mg |

| OPTIONAL COATING B (inert water soluble lamina) | |
|---|---|
| Water soluble polymer | 1.00-15.00 mg |
| Opaquant | 1.00-20.00 mg |
| Antiadherent | 1.00-25 mg |
| Colorant | 0.1-1.00 mg |

The ranges are proportional to the 150 and 600 mg strengths.

First, the core composition is prepared by placing amantadine HCl, an osmagent, a diluent and a binder in a high shear mixer and mix. The granulation process is initiated by the gradual addition of a granulating solution containing a plasticizer and purified water to the high shear with continuous blending to produce a wet blend. Next, the wet blend is granulated and dried in a fluid bed to remove the water. Then, the dry granules are screened through an USP mesh screen for size reduction. Next, the screened granules are mixed with a glidant and a lubricant. This final blend is tabletted to provide the cores.

A first composition to cover the coated cores is prepared as follows: cellulose ester and a plastizicer are added to a solvent or suspending solution and mixed thoroughly to form a polymer-solution. This solution is sprayed onto the tablets in a perforated pan coater to form semipermeable membrane coated cores. At least one hole is drilled through the coating to provide perforated osmotic cores.

An optional second composition to cover the perforated osmotic cores is prepared as follows: a water soluble polymer, an opaquant, an antiadherent and a colorant are mixed in a solvent. This polymer mixture is sprayed onto the perforated osmotic cores to obtain film-coated tablets.

The osmagent, diluent, binder, plasticizer, glidant, lubricant, cellulose ester, water soluble polymer,disintegrant, opaquant, and film forming polymer used in the present formulation were selected from the respective groups of ingredients set forth above.

### EXAMPLE 2

### Osmotic device containing AMN in the core and CIT in a surrounding coating

The following procedure is used to prepare multi-layered osmotic device tablets containing amantadine HCl (150, 300 and 600 mg strength) in the osmotic core and citalopram HBr (5, 10 and 20 mg strength) in a drug-containing external coat of the osmotic device. The osmotic device tablets prepare according to the method disclosed in Example 1 are coated with a citalopram-containing composition and a finish coat containing the following ingredients in the amounts indicated:

| **INGREDIENT** | **AMOUNT (MG)** |
|---|---|
| Amantadine Strength⇒ | 300.0 |
| Citalopram Strengths ⇒ | 10.0 |

| **COATING C** | |
|---|---|
| Citalopram Hydrobromide | 12.49 |
| Water Soluble Film Polymer | 1.80-7.52 |
| Disintegrant | 0.85-3.70 |
| Plasticizer | 0.50-3.50 |

| **COATING D** | |
|---|---|
| Opadry 1 | 10-30 |
| Purified Water* | 105-230 |

The ranges are proportional to the 5 and 20 mg strengths.

A coating C composition to cover the osmotic tablets of example 1 is prepared as follows: citalopram hydrobromide, water soluble film polymer, desintegrant, and plasticizer are added to the purified water to form the coating suspension. This suspension is sprayed onto the tablets in a perforated pan coater to obtain drug load coated tablets.

A finish coat, coating D, comprising Opadry in purified water is applied onto the film-coated tablets to obtain the multi-layered osmotic device tablets.

The osmagent, diluent, binder, plasticizer, glidant, lubricant, cellulose ester, water soluble polymer,disintegrant, opaquant, and film forming polymer used in the present formulation were selected from the respective groups of ingredients set forth above.

### EXAMPLE 3

### Osmotic device containing AMN and CIT in the core

The following procedure is used to prepare osmotic device tablets containing amantadine (150, 300 and 600 mg strength) and citalopram (5, 10 and 20 mg strength) in admixture in the core of the osmotic device. The osmotic device tablets contain the following ingredients in the amounts indicated:

| **INGREDIENT** | **AMOUNT (mg)** | | |
|---|---|---|---|
| **Amantadine Strength⇒** | 150.0 | 300.0 | 600.0 |
| **Citalopram Strength⇒** | 20.0 | 10.0 | 5.0 |

| **CORE** | | | |
|---|---|---|---|
| Amantadine Hydrochloride | 150.00 | 300.00 | 600.00 |
| Citalopram Hydrobromide | 24.98 | 12.49 | 6.25 |
| Sodium Chloride | 37.00 | 70.00 | 125.00 |
| Hydroxypropyl methylcellulose 2208 | 4.20 | 8.1 | 15.80 |
| Polyethylene Oxide | 20.00 | 38.00 | 64.50 |
| Povidone | 4.00 | 8.00 | 16.00 |
| Polyethylene Glycol 400 | 2.00 | 4.00 | 8.00 |
| Cellulose Microcrystalline | 5.82 | 33.41 | 220.45 |
| Colloidal Silicon Dioxide | 0.50 | 1.00 | 2.00 |
| Magnesium Stearate | 1.50 | 3.00 | 6.00 |

| **COATING A** | | | |
|---|---|---|---|
| Cellulose Acetate 320 | 9.60 | 11.78 | 21.22 |
| Cellulose Acetate 398 | 8.50 | 11.05 | 18.78 |
| Polyethylene Glycol 400 | 0.90 | 1.17 | 2.00 |

| **COATING B** | | | |
|---|---|---|---|
| Opadry 1 | 12.00 | 20.00 | 30.00 |
| Purified Water* | 108.00 | 180.00 | 270.00 |

First, the core composition is prepared by placing amantadine HCl, citalopram HBr, sodium chloride, microcrystalline cellulose, HPMC 2208, polyethylene oxide (MW 600.000) and povidone in a high shear mixer and mixing for 5 minutes. The granulation process is initiated by the gradual addition of a granulating solution containing polyethylene glycol 400 and purified water to the high shear with continuous blending to produce a wet blend. Next, the wet blend is granulated and dried at 45-55°C for 30 minutes in a fluid bed to remove the water. Then, the dry granules are screened through a 30 USP mesh screen for size reduction. Next, the screened granules are mixed with the colloidal silicon dioxide and magnesium stearate, that have been previously passed through a 60 mesh screen, in a V-Blender during 5 minutes. This final blend is tabletted to provide the cores.

A first composition to cover the coated cores is prepared as follows: cellulose acetate and polyethylene glycol 400 are added to acetone and purified water and mixed thoroughly to form a polymer solution. This polymer solution is sprayed onto the tablets in a perforated pan coater to form semipermeable membrane coated cores. A 0.5 mm hole is drilled through the coating to provide perforated cores.

A finish coat comprising Opadry in purified water is applied onto the film-coated tablets to obtain the multi-layered osmotic device tablets.

### EXAMPLE 4

### Osmotic Device containing AMN in the core and BUS in a surrounding coating

The following procedure is used to prepare multi-layered osmotic device tablets containing amantadine HCl (150, 300 and 600 mg strength) in the osmotic core and buspirone HCl (5, 7.5 and 15 mg strength) in a drug-containing external coat of the osmotic device. The osmotic device tablets contain the following ingredients in the amounts indicated:

| **INGREDIENT** | **AMOUNT (mg)** | | |
|---|---|---|---|
| **Amantadine Strength⇒** | 150.0 | 300.0 | 600.0 |
| **Buspirone Strength⇒** | 15.00 | 7.50 | 5.00 |

| **CORE** | | | |
|---|---|---|---|
| Amantadine Hydrochloride | 150.00 | 300.00 | 600.00 |
| Mannitol | 20.00 | 40.00 | 80.00 |
| Copolyvidone | 4.00 | 8.00 | 16.00 |
| Polyethylene Glycol 400 | 3.00 | 6.00 | 12.00 |
| Cellulose Microcrystalline | 11.00 | 22.00 | 33.00 |
| Colloidal Silicon Dioxide | 0.50 | 1.00 | 2.00 |
| Magnesium Stearate | 1.50 | 3.00 | 6.00 |

| **COATING A** | | | |
|---|---|---|---|
| Cellulose Acetate 398 | 15.30 | 23.75 | 36.10 |
| Polyethylene Glycol 400 | 0.70 | 1.25 | 1.90 |

| **COATING B** | | | |
|---|---|---|---|
| Buspirone Hydrochloride | 15.00 | 7.50 | 5.00 |
| 2910 Hydroxypropyl methylcellulose | 8.40 | 4.20 | 2.80 |
| Crospovidone | 3.00 | 1.50 | 1.00 |
| Polyethylene Glycol 400 | 1.60 | 0.80 | 1.20 |

| **COATING C** | | | |
|---|---|---|---|
| Opadry 1 | 12.00 | 20.00 | 25.00 |
| Purified Water* | 108.00 | 180.00 | 225.00 |

First, the core composition is prepared by placing amantadine HCl, mannitol, microcrystalline cellulose and copolyvidone in a high shear mixer and mix for 5 minutes. The granulation process is initiated by the gradual addition of a granulating solution containing polyethylene glycol 400 and purified water to the high shear with continuous blending to produce a wet blend. Next, the wet blend is granulated and dried at 40-50°C for 20 minutes in a fluid bed to remove the water. Then, the dry granules are screened through a 30 USP mesh screen for size reduction. Next, the screened granules are mixed with the colloidal silicon dioxide and magnesium stearate, that have been previously passed through a 60 mesh screen, in a V-Blender during 5 minutes. This final blend is tabletted to provide the cores.

A first composition to cover the coated cores is prepared as follows: cellulose acetate and polyethylene glycol 400 are added to acetone and mixed thoroughly to form a polymer solution. This solution is sprayed onto the tablets in a perforated pan coater to form semipermeable membrane coated cores. A 0.5 mm hole is drilled through the coating to provide perforated cores.

A second composition to cover the perforated cores is prepared as follows: buspirone HCL, HPMC 2910, crospovidone and polyethylene glycol 400 are added to the acetone to form the coating suspension. This suspension is sprayed onto the tablets in a perforated pan coater to obtain drug load coated tablets.

A finish coat comprising Opadry in purified water is applied onto the film-coated tablets to obtain the multi-layered osmotic device tablets.

### EXAMPLE 5

### Osmotic device containing AMN and BUS in the core

The following procedure is used to prepare osmotic device tablets containing amantadine (150, 300 and 600 mg strength) and buspirone (5, 7.5 and 15 mg strength) in admixture in the core of the osmotic device. The osmotic device tablets contain the following ingredients in the amounts indicated:

| **INGREDIENT** | **AMOUNT (mg)** | | |
|---|---|---|---|
| **Amantadine Strength⇒** | 150.0 | 300.0 | 600.0 |
| **Buspirone Strength⇒** | 5.00 | 7.50 | 10.00 |

| **CORE** | | | |
|---|---|---|---|
| Amantadine Hydrochloride | 150.00 | 300.00 | 600.00 |
| Buspirone Hydrochloride | 5.00 | 7.50 | 15.00 |
| Sodium Chloride | 21.00 | 42.00 | 84.00 |
| Sodium Carboxymethylcellulose | 3.00 | 6.00 | 12.00 |
| Polyethylene Glycol 400 | 2.00 | 4.00 | 8.00 |
| Cellulose Microcrystalline | 7.00 | 16.50 | 33.00 |
| Colloidal Silicon Dioxide | 0.50 | 1.00 | 2.00 |
| Magnesium Stearate | 1.50 | 3.00 | 6.00 |

| **COATING A** | | | |
|---|---|---|---|
| Cellulose Acetate 320 | 7.00 | 11.00 | 16.00 |
| Cellulose Acetate 398 | 4.10 | 4.90 | 2.00' |
| Polyethylene Glycol 400 | 0.90 | 1.10 | 2.00 |

| **COATING B** | | | |
|---|---|---|---|
| Opadry 1 | 12.00 | 20.00 | 30.00 |
| Purified Water* | 108.00 | 180.00 | 270.00 |

First, the core composition is prepared by placing amantadine HCl, buspirone HCl, sodium chloride, microcrystalline cellulose, and sodium carboxymethylcellulose in a high shear mixer and mix for 5 minutes. The granulation process is initiated by the gradual addition of a granulating solution containing polyethylene glycol 400 and purified water to the high shear with continuous blending to produce a wet blend. Next, the wet blend is granulated and dried at 45-55°C for 30 minutes in a fluid bed to remove the water. Then, the dry granules are screened through a 30 USP mesh screen for size reduction. Next, the screened granules are mixed with the colloidal silicon dioxide and magnesium stearate, that have been previously passed through a 60 mesh screen, in a V-Blender during 5 minutes. This final blend is tabletted to provide the cores.

A first composition to cover the coated cores is prepared as follows: cellulose acetate and polyethylene glycol 400 are added to acetone and mixed thoroughly to form a polymer solution. This polymer solution is sprayed onto the tablets in a perforated pan coater to form semipermeable membrane coated cores. A 0.5 mm hole is drilled through the coating to provide perforated cores.

A finish coat comprising Opadry in purified water is applied onto the film-coated tablets to obtain the osmotic device tablets.

### EXAMPLE 6

### Osmotic device containing AMN and VFX in the core

The following procedure is used to prepare osmotic device tablets containing amantadine (150, 300 and 600 mg strength) and venlafaxine (150, 75 and 37.5 mg strength) in admixture in the core of the osmotic device. The osmotic device tablets contain the following ingredients in the amounts indicated:

| **INGREDIENT** | **AMOUNT (mg)** | | |
|---|---|---|---|
| **Amantadine Strengt⇒** | 150.0 | 300.0 | 600.0 |
| **Venlafaxine Strength⇒** | 150.0 | 75.0 | 37.5 |

| **CORE** | | | |
|---|---|---|---|
| Amantadine Hydrochloride | 150.00 | 300.00 | 600.00 |
| Venlafaxine Hydrochloride | 169.72 | 84.86 | 37.72 |
| Mannitol | 26.28 | 47.14 | 94.28 |
| Sodium Carboxymethylcellulose | 3.00 | 6.00 | 12.00 |
| Polyethylene Glycol 400 | 2.00 | 4.00 | 8.00 |
| Cellulose Microcrystalline | 7.00 | 14.00 | 28.00 |
| Colloidal Silicon Dioxide | 0.50 | 1.00 | 2.00 |
| Magnesium Stearate | 1.50 | 3.00 | 6.00 |
| Purified water* | 15.00 | 20.00 | 25.00 |

| **COATING A** | | | |
|---|---|---|---|
| Cellulose Acetate 398 | 11.10 | 15.90 | 18.00 |
| Polyethylene Glycol 400 | 0.90 | 1.10 | 2.00 |
| Acetone* | 330.40 | 429.50 | 730.20 |

| **COATING B** | | | |
|---|---|---|---|
| Opadry 1 | 15.00 | 20.00 | 25.00 |
| Purified Water* | 135.00 | 180.00 | 225.00 |

| | | | |
|---|---|---|---|
| * Indicates liquids used to make the tablets, but the liquids are for the most part absent from the final tablet. | | | |

First, the core composition is prepared by placing amantadine HCl, venlafaxine HCl, mannitol, microcrystalline cellulose, and sodium carboxymethylcellulose in a high shear mixer and mix for 5 minutes. The granulation process is initiated by the gradual addition of a granulating solution containing polyethylene glycol 400 and purified water to the high shear with continuous blending to produce a wet blend. Next, the wet blend is granulated and dried at 45-55°C for 30 minutes in a fluid bed to remove the water. Then, the dry granules are screened through a 30 USP mesh screen for size reduction. Next, the screened granules are mixed with the colloidal silicon dioxide and magnesium stearate, that have been previously passed through a 60 mesh screen, in a V-Blender during 5 minutes. This final blend is tabletted to provide the cores.

A first composition to cover the coated cores is prepared as follows: cellulose acetate and polyethylene glycol 400 are added to acetone and mixed thoroughly to form a polymer solution. This polymer solution is sprayed onto the tablets in a perforated pan coater to form semipermeable membrane coated cores. A 0.5 mm hole is drilled through the coating to provide perforated cores.

A finish coat comprising Opadry in purified water is applied onto the film-coated tablets to obtain the osmotic device tablets.

### EXAMPLE 7

### Matrix tablet containing AMN and CIT

The following procedure is used to prepare matrix tablets containing amantadine (150, 300 and 600 mg strength) and citalopram (5, 10 and 20 mg strength). The tablets contain the following ingredients in the amounts indicated:

| **INGREDIENT** | **AMOUNT (mg)** | | |
|---|---|---|---|
| **Amantadine Strength⇒** | 150.0 | 300.0 | 600.0 |
| **Citalopram Strength⇒** | 20.0 | 10.0 | 5.0 |

| **CORE** | | | |
|---|---|---|---|
| Amantadine Hydrochloride | 150.00 | 300.00 | 600.00 |
| Citalopram Hydrobromide | 24.98 | 12.49 | 6.25 |
| HPMC 2208 4000 cps | 5.00 | 10.00 | 20.00 |
| HPMC 2208 100.000 cps | 10.02 | 20.02 | 40.02 |
| Cellulose Microcrystalline | 2.00 | 41.49 | 101.73 |
| Copolyvidone | 5.00 | 10.00 | 20.00 |
| Polyethylene Glycol 400 | 1.00 | 2.00 | 4.00 |
| Colloidal Silicon Dioxide | 0.50 | 1.00 | 2.00 |
| Magnesium Stearate | 1.50. | 3.00 | 6.00 |
| Purified water* | 16.00 | 30.00 | 60.00 |

First, the core composition is prepared by placing amantadine HCl, citalopram hydrobromide, mannitol, microcrystalline cellulose, hydroxypropyl methylcellulose 2208 4000 cps, hydroxypropyl methylcellulose 2208 100.000 cps and copolyvidone in a high shear mixer and mix for 5 minutes. The granulation process is initiated by the gradual addition of a granulating solution containing polyethylene glycol 400 and purified water to the high shear with continuous blending to produce a wet blend. Next, the wet blend is granulated and dried at 45-55°C for 30 minutes in a fluid bed to remove the Water. Then, the dry granules are screened through a 30 USP mesh screen for size reduction. Next, the screened granules are mixed with the colloidal silicon dioxide and magnesium stearate, that have been previously passed through a 60 mesh screen, in a V-Blender during 5 minutes. This final blend is tabletted to provide the matrix tablets.

### EXAMPLE 8

### Determination of clinical effect

The combination of amantadine with and SSRI, SNRI or anxiolytic agent is an efficacious combination for reducing L-dopa induced dyskinesias, and for potentiating the therapeutic effect of L-dopa. The clinical effect of these combinations is characterized on the following clinical endpoints in patients with Parkinson's Disease: 1) UPDRS part III (motor examination), 2) UPDRS part IV, items 32 - 35 (presence and severity of dyskinesias), 3) per diary record the average duration of time in "off" and "on" states, and 4) the average daily dose of L-Dopa.

### EXAMPLE 9

### Method of treating subjects with Parkinson's disease

Patients with Parkinson's Disease are dosed as follows. In specific embodiments, either AMN or MEM are used as the NMDA-RA, and either CIT, BUS or TRA are used as the neuroactive agent.

Method A: Patients are dosed once a day with a combination of amantadine and an SSRI, SNRI or anxiolytic agent on top of their L-dopa regimen. Patients will have been on L-dopa regimen prior to the dosing with amantadine combination. Patients are characterized according to: 1) UPDRS part III (motor examination), 2) UPDRS part IV, items 32 - 35 (presence and severity of dyskinesias), 3) per diary record the average duration of time in "off" and "on" states; and 4) the average daily dose of L-dopa prior to dosing with amantadine combination for determining the baseline disease level. After 4 weeks of dosing with the amantadine combination on top of the L-dopa regimen, patients will improve on one or more of the four endpoints.

One embodiment of a method of treating subjects with Parkinson's disease is generally described as follows. Patients with Parkinson's Disease that are undergoing L-Dopa therapy are dosed once daily with a combination of amantadine and an SSRI, SNRI or anxiolytic agent on top of their L-dopa regimen. In other words, the L-Dopa is generally administered in a dosage form separate from the one of the invention. The dosage regimen for amantadine is ranged from 50 to 800 mg daily according to each patient response. The dosage regimen for the SSRI, the SNRI or the anxyolitic agent is ranged from subtherapeutic doses, such as 5 mg for citalopram, 12 mg for venlafaxine and 25 mg for buspirone to current therapeutic doses, 40 mg for citalopram, 40 mg for buspirone and 150 mg for venlafaxine. Each drug is titrated according to the level of reduction of dyskinesias, reduction of Parkinsonism effects, and production of neuroprotectant activities. Thus adequate dosage regimen is adjusted for each individual according to the level of response on the endpoints mentioned below. Patients are characterized according to: 1) UPDRS part III (motor examination); 2) UPDRS part IV, items 32 - 35 (presence and severity of dyskinesias); 3) diary record of the average duration of time in "off' and "on" states; 4) the average daily dose of L-dopa prior to dosing with amantadine combination for determining the baseline disease level; 5) Hamilton Rating Scale for depression (HAM-D) and Beck Depression Inventory (BDI); 6) Alzheimer s Disease Assessment Scale-Cognitive Subscale (ADAS-Cog) and Clinician Interview based Impression of Change plus Caregiver Input (CIBIC plus). Improvement in points 1 to 4 appear in the case of motor fluctuations and dyskinesias induced by antiparkinsonian therapy; and improvement in points 5-6 are observed in the case of neuroprotection and antiparkinsonian effects after a period ranging from 4 to 18 weeks of dosing with the drug combination.

Method B: Patients are dosed with a combination of amantadine and citalopram to demonstrate a superior efficacy of the combined administration of amantadine and citalopram compared with amantadine and placebo in either the improvement of motor performance - particularly bradykinesia - and/or in the reduction of motor fluctuations - particularly dyskinesias - induced by L-DOPA therapy, in Parkinson Disease patients. The study is conducted in a double-blind, randomized, placebo-controlled, parallel-group design, with 2 treatment phases: 3 weeks open label, up -titration amantadine lead-in phase, follow by a 6 weeks add-on citalopram phase; performed in Parkinson Disease patients suffering from severe bradykinesia and motor fluctuations -dyskinesias- induced by L-Dopa therapy.

A total number of 104 patients are enrolled in 4 study centers, to ensure that 80 patients will complete the study treatment according to the following protocol:

### Patient Population

Male and female outpatients, aged 30 to 75 years, with a diagnosis of idiopathic Parkinson's Disease presenting severe bradykinesia and motor fluctuations-dyskinesias-induced by levodopa therapy

### Inclusion Criteria

- Parkinson Disease duration of no less than 5 years, onset of symptoms of more than 1 year, and onset of bradykinesia symptom of more than 1 year.
- Patients with Hoehn and Yahr stage II to IV, and patients having received L-Dopa/DCCI treatment for no less than 2 years.
- Patients presenting body bradykinesia with a UPDRS score item 31 from 2 to 4.
- Cognitive-intact patients, as determined by a score of greater than 26 on the MiniMental Score status examination, and able to execute written informed consent.
- The physician and the patient must be willing to defer planned reductions of L-Dopa dosage until after completion of the study.

### Exclusion criteria

- Patients with a diagnosis of secondary parkinsonian syndromes, such as vascular, post-inflammatory, drug-induced, neoplastic and post-traumatic, and taking any other antiparkinsonian therapy except for L-dopa/DDC inhibitors combination therapy.
- Patients with a diagnosis of mayor depressive disorder (DSM-IV) within the past 12 months.
- Pregnant or lactating patients, and patients enrolled in other protocols involving investigational drugs or surgery.
- Hypersensitivity to any of the investigational drugs, based on known allergies to drugs of the same class.
- Ingestion of any investigational medication within 30 days prior to first dose of study medication.

### Dosage Schedule

The following graphic shows the 2 different arms 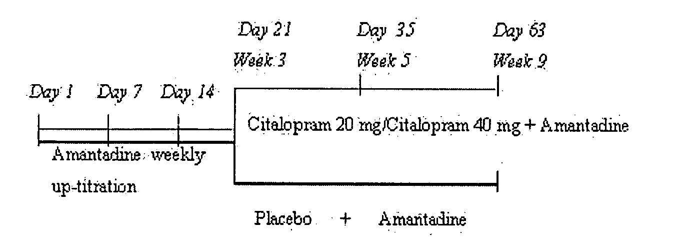
Group 1 : Open label Amantadine + Citalopram arm
Group 2 : Open label Amantadine + Placebo arm

### Administration

### Group 1: Open label Amantadine + Citalopram arm

### Lead-in, open label, up-titration amantadine period: (Weeks 1, 2 and 3):

Amantadine Hydrochloride: 50 mg amantadine hydrochloride is administered as a b.i.d. regimen during 1 week. Up titration to 100 mg amantadine hydrochloride administered as a b.i.d. regimen during the second week. Up titration to 150 mg amantadine hydrochloride administered as a b.i.d. regimen during the third week.

### Add-on, randomized, double blind period: (Weeks 4 to 9):

Citalopram: 20-mg citalopram is administered as a q.d. regimen during 3 weeks plus 1 placebo capsule administered daily, as a q.d. regimen for blinding purposes. Up-titration to 40 mg citalopram is administered as a q.d. regimen during 1 week plus 1 placebo capsule administered daily, as a q.d. regimen for blinding purposes. During add-on period, open label amantadine should be continued up to week 9, with the highest dose reached in the lead-in phase. Down or up titration during add-on phase is not accepted

### Group 2: Open label Amantadine + Placebo arm

### Lead-in, open label, up-titration amantadine period: (Weeks 1, 2 and 3)

Amantadine Hydrochloride: 50 mg amantadine hydrochloride is administered as a b.i.d. regimen during 1 week. Up titration to 100 mg amantadine hydrochloride administered as a b.i.d. regimen during the second week. Up titration to 150 mg amantadine hydrochloride administered as a b.i.d. regimen during the third week.

### Add-on, randomized, double blind period:

### 2 placebo capsule are administered daily as a b.i.d. regimen for blinding purposes

Open label amantadine is continued up to week 9, with the highest dose reached in the lead-in phase. Down or up titration during add-on phase is not accepted.

### Amantadine up and down titration criteria :

Efficacy criteria: amantadine is titrated during the 3 weeks, lead-in phase, up to the necessary dose for obtaining an improvement of 40% in UPDRS III and/or IV scores compared with baseline score , or if an improvement of 40% is not obtained, weekly titration is done up to 150 mg b.i.d .

Safety criteria: if an adverse event with any relationship to amantadine occurred, down titration during lead-in period is done, up to 50 mg b.i.d. If the adverse event persists, patient are retrieved from the trial. Down titration during double blind phase is not accepted.

### Efficacy Evaluations

a) The Unified Parkinson's Disease Rating Scale (UPDRS) is used as an overall assessment scale to quantify the motor state and presence and severity of dyskinesias. UPDRS is composed of 4 parts:
   The first part evaluates: mentation, behavior and mood, and includes evaluation of intellectual impairment, thought disorder, depression and motivation.
   The second part evaluates activities of daily living and includes evaluation of speech, salivation, swallowing, handwriting, cutting food and handling utensils, dressing, hygiene, turning in bed and adjusting bed clothes, falling, freezing when walking, walking, tremor and sensory complaints related to parkinsonism.
   The third part evaluates motor activities and includes evaluation of speech, facial expression, tremor at rest, action or postural tremor of hands, rigidity, finger taps, hand movements, rapid alternating movements of hands, leg agility, arising from chair, posture, gait, postural stability, body bradykinesia and hypokinesia.
   The fourth part evaluates complications of therapy during the previous week of evaluation and includes evaluation of dyskinesias, clinical fluctuations and other complications.
   Each evaluation is numbered assigned so that a total of 40 points are evaluated in this scale. Each point is scored from 0, meaning the less severe, to 4 meaning the most severe score.
   The total score for UPDRS subscale IV is employed to evaluate the severity and characteristics of L-Dopa induced dyskinesias. The total score is calculated as the sum of all scores assigned in the dyskinesia items: 32, 33, 34 and 35; the clinical fluctuations items (calculated as the sum of all scores assigned in items 36, 38 and 39). Items 32, 33, 34 and 35 are recorded according to historical information and item 33 is also modified by office examination. Total score range is from 0 to 13 for dyskinesias and from 0 to 7 for motor fluctuations.
   The total score of the subscale III of the UPDRS is employed to examine parkinsonian symptoms. Motor functionality is assessed as the total motor score calculated as the sum of all scores assigned in items 18, 19, 20, 21, 22, 23, 24, 25, 26, 27, 28, 29, 30 and 31. Total motor score value will range from 0 to 56, being the zero the less severe and fifty six the most severe profile for the parkinsonian symptoms.
   The subscale III of the UPDRS is assessed during both "ON" and "OFF" periods. Separate scores are obtained for each assessment.
   Item 31 of the subscale III of the UPDRS is individually evaluated to assess bradykinesia (combining slowness, hesitancy, decreased arm swing, small amplitude, and poverty of movement in general). The score assigned by the physician in item 31 will considered 0 = none, 1= minimal, 2= mild, 3= moderate and 4= marked slowness, poverty or small amplitude of movement.
   Item 23 of the subscale III of the UPDRS will evaluate finger taps and a four points scale is used to evaluate it, where 0=normal, 1=mild slowing and/or reduction in amplitude, 2=moderate impaired, 3=severely impaired and 4=can barely perform the task.
   Subscale I of UPDRS is used to evaluate mentation, behavior and mood; it is a four items scale, with valuation of intellectual impairment, thought disorders, depression and motivation, each item has a four point evaluation scale.
   Subscale II of UPDRS that evaluate activities of daily living and is used for both "on" and "off" periods. This scale has 13 items, each one with 4 points evaluation scale; the items are: speech, salivation, swallowing, handwriting, cutting food and handling utensils, dressing, hygiene, turning in bed and adjusting bed clothes, falling, freezing with walking, walking, tremor and sensory complaints.
   Subscales I and II are used to evaluate secondary endpoints.
b) The Abnormal Involuntary Movement Scale (AIMS) is a clinical rating instrument that measures dyskinesias. It includes individual body areas scales that assess the severity of movement impairment in different body areas, such as facial, oral, limbs and trunk movements. The physician will assign a five point score for each area, considering 0 = none, 1= minimal, 2= mild, 3= moderate and 4=severe. A total scale that results from the addition of individual body areas ratings is calculated.
c) Mobility state self-assessment. Patients are instructed to perform the mobility state self-assessment status every two hours during the waking time for the previous two days of each study visit. Patients are given individual diary forms and by means of symbols, will have to note down three different possible-states: good mobility ("ON"), rigidity or poor mobility ("OFF") or involuntary movements ("DYSKINESIAS"). A total value of hours is calculated as the sum of hours during waking time for each state at the end of each study period.
d) BRAIN TEST: is a software program, which is based on the alternating finger taping test. It uses a standard personal computer with the keyboard as the test device. The two targets are the "S" and ";" keys which are 15 cm apart on the 101/102 keyboards, as well as the keyboards on most notebook size computers.
   The target keys are marked with adhesive red paper dots 10 mm in diameter. Test subjects are seated confortably in front of the keyboard at a height that allows their arms to be above the keyboard when their elbows are flexed at 90°. Using the index finger the subject has to alternatively strike the target keys for a period of 60 seconds. Before starting the tests the subjects are told to perform the test as fast and accurately as possible. The data generated are analyzed to produce several variables. The test is performed on both upper limbs.
e) The Folstein Mini-Mental Status Exam (MMSE). The Mini-Mental State Examination is a brief, quantitative measure of cognitive mental status in adults. It is used to screen for cognitive impairment and to estimate the severity of this impairment. It contains a series of questions that evaluates memory, language, calculation and orientation. A 30 point scale is used. MMSE scores under 24 is indicative of significant impairment; patients are not included in the study protocol. The performance of the minimental examination includes a series of question that the investigator performes on the patient in order to define the MMSE score on entry.
f) DSM -IV TR (Criteria for Major Depressive Episode).
g) Modified Hoehn and Yahr Staging.

### Safety Evaluations

Safety is evaluated on the basis of periodical anamnesis and physical examination. Patients are monitored for general well being and adverse events throughout the duration of the study, as they occurred.

### Statistical Analysis

Non parametric techniques for independent and paired multiple samples: Kruskal Wallis test and Friedman test.

### EXAMPLE 10

### Capsule containing Amantadine (CR) and Citalopram (IR)

The following procedure is used to prepare capsules containing Amantadine HCl (150, 300 and 600 mg strength) and Citalopram HBr (5, 10 and 20 mg strength). The capsules contain the following ingredients in the amounts indicated:

| **INGREDIENT** | **AMOUNT (mg)** | | |
|---|---|---|---|
| **Amantadine Strength⇒** | 150.0 | 300.0 | 600.0 |
| **Citalopram Strength⇒** | 20.0 | 10.0 | 5.0 |

| **CORE** | | | |
|---|---|---|---|
| Amantadine Hydrochloride | 150.00 | 300.00 | 600.00 |
| Lactose Monohydrate | 10.00 | 15.00 | 20.00 |
| Ethylcellulose | 4.50 | 6.20 | 8.00 |
| Polyethylene Glycol 400 | 0.50 | 0.80 | 1.00 |
| Acetone | 50.00 | 100.00 | 150.00 |
| Citalopram HBr | 24.98 | 12.49 | 6.25 |
| Sugar | 2.00 | 4.00 | 8.00 |
| Talcum | 0.50 | 1.00 | 1.50 |
| Dried Starch | 7.52 | 10.51 | 5.75 |
| Purified water* | 50.00 | 100.00 | 150.00 |
| Capsule weight | 200.00 | 350.00 | 650.00 |

A first microgranulate (group of microgranules) is prepared as follows. Amantadine HCl and lactose monohydrate are mixed for 5 minutes in a fluid bed. Ethylcellulose, polyethylene glycol 400 are added to acetone to form a solution. This solution is sprayed onto the granules in a fluid bed to obtain coated microgranules.

A second microgranulate is prepared as follows. Citalopram hydrobromide and and dried starch are mixed in a fluid bed for 5 minutes. Sugar and Talcum are added to purified water to form a coating suspension. This suspension is sprayed onto the granules in a fluid bed to obtain coated microgranules.

Both microgranulates are placed into appropriately sized capsule shells. The weight ratio of first to second microgranulates in this example is about 1:1; however, the ratio can be adjusted as needed to provide a capsule dosage form with the appropriate strengths of active agents.

### EXAMPLE 11

The following procedure is used to prepare multi-layered osmotic device tablets containing venlafaxine (37.5, 75 and 150 mg strength) in the core and memantine (10 and 40 mg strength) in a drug-containing external coat of the osmotic device. The venlafaxine is released in a controlled manner and the memantine is released in a rapid manner. The osmotic device tablets contain the following ingredients in the amounts indicated.

| **INGREDIENT** | **AMOUNT (mg)** | | | | | |
|---|---|---|---|---|---|---|
| **Venlafaxine Strength⇒** | 37.5 | 37.5 | 75 | 75 | 150 | 150 |
| **Memantine Strength⇒** | 10 | 40 | 10 | 40 | 10 | 40 |

| **CORE** | | | | | | |
|---|---|---|---|---|---|---|
| Venlafaxine Hydrochloride | 42.43 | 42.43 | 84.86 | 84.86 | 169.72 | 169.72 |
| Mannitol | 25.00 | 25.00 | 50.00 | 50.00 | 100.00 | 100.00 |
| Povidone k-90 | 3.50 | 3.50 | 7.00 | 7.00 | 14.00 | 14.00 |
| Polyethylene Glycol 400 | 2.50 | 2.50 | 5,00 | 5.00 | 10.00 | 10.00 |
| Cellulose Microcrystalline | 14.57 | 14.57 | 29.14 | 29.14 | 58.28 | 58.28 |
| Colloidal Silicon Dioxide | 0.50 | 0.50 | 1.00 | 1.00 | 2.00 | 2.00 |
| Magnesium Stearate | 1.50 | 1.50 | 3.00 | 3.00 | 6.00 | 6.00 |
| Purified water* | 15.00 | 15.00 | 30.00 | 30.00 | 60.00 | 60.00 |

| **COATING A** | | | | | | |
|---|---|---|---|---|---|---|
| Cellulose Acetate 398 | 7.88 | 7.88 | 15.77 | 15.77 | 31.54 | 31.54 |
| Polyethylene Glycol 400 | 0.42 | 0.42 | 0.83 | 0.83 | 1.66 | 1.66 |
| Acetone* | 130.37 | 130.37 | 260.74 | 260.74 | 521.48 | 521.48 |

| **COATING B** | | | | | | |
|---|---|---|---|---|---|---|
| Memantine Hydrochloride | 12.03 | 48.13 | 12.03 | 48.13 | 12.03 | 48.13 |
| HPMC 2910 | 1.47 | 5.87 | 3.97 | 15.87 | 6.97 | 27.87 |
| Crospovidone | 0.50 | 2.00 | 2.00 | 8.00 | 2.50 | 10.00 |
| Polyethylene Glycol 400 | 1.00 | 4.00 | 2.00 | 8.00 | 3.50 | 14.00 |
| Acetone* | 120.00 | 480.00 | 160.00 | 640.00 | 200.00 | 800.00 |

| **COATING C** | | | | | | |
|---|---|---|---|---|---|---|
| Opadry 1 | 10.00 | 10.00 | 15.00 | 15.00 | 20.00 | 20.00 |
| Purified Water* | 100.00 | 100.00 | 150.00 | 150.00 | 200.00 | 200.00 |

The core composition is prepared by placing venlafaxine, mannitol, cellulose microcrystalline and half the quantity of colloidal silicon dioxide in a high shear mixer and mixing for 3 minutes. The granulation process is initiated by the gradual addition of a granulating fluid comprising polyethylene glycol 400, povidone, and purified water to the mixer with continuous mixing to produce a wet blend. The wet blend is granulated and dried at 40-50°C for 15 minutes in a fluid bed to remove the granulating fluid. The dry granules are screened through a mesh screen for size reduction. The screened granules are mixed with the rest of colloidal silicon dioxide, that has been previously passed through a 60 mesh screen, and mixed with magnesium stearate, that has been previously passed through a 60 mesh screen. This final blend is tabletted to provide the cores.

A first composition to cover the coated cores is prepared as follows. Cellulose acetate 398 and polyethylene glycol 400 are added to acetone and mixed thoroughly to form a polymer mixture. This polymer mixture is sprayed onto the tablets in a perforated pan coater to form semipermeable membrane coated cores. A 0.5 mm hole is drilled through the coating to provide perforated cores. This first coating composition can also be manufactured with the ingredients in the amounts indicated in the table below as follows. Cellulose acetate 398, cellulose acetate 320 and polyethylene glycol 400 are added to a blend of methylene chloride, methanol and acetone, and mixed thoroughly to form a polymer mixture that is sprayed onto the tablets. A 0.5 mm hole is drilled through the coating.

| **COATING A** | | | | | | |
|---|---|---|---|---|---|---|
| Cellulose Acetate 398 | 8.02 | 8.02 | 16.03 | 16.03 | 32.06 | 32.06 |
| Cellulose Acetate 320 | 2.11 | 2.11 | 4.22 | 4.22 | 8.44 | 8.44 |
| PEG 400 | 0.52 | 0.52 | 1.05 | 1.05 | 2.10 | 2.10 |
| Methylene Chloride | 5.91 | 5.91 | 11.82 | 11.82 | 23.64 | 23.64 |
| Methanol | 120.07 | 120.07 | 140.14 | 140.14 | 280.28 | 280.28 |
| Acetone | 50.00 | 50.00 | 100.00 | 100.00 | 200.00 | 200.00 |
| Purified water | 21.10 | 21.10 | 42.20 | 42.20 | 84.40 | 84.40 |

A second composition to cover the perforated cores is prepared as follows. Memantine HCl, HPMC 2910, crospovidone and polyethylene glycol 400 are added to the acetone to form a polymer mixture. This polymer mixture is sprayed onto the tablets in a perforated pan coater to obtain film-coated tablets.

A finish coat comprising Opadry in purified water is applied onto the film-coated tablets to obtain the multi-layered osmotic device tablets.

### EXAMPLE 12

### Matrix tablet containing NMDA-RA and Neuroactive Agent

The procedure of Example 7 is used to prepare matrix tablets, containing an NMDA-RA and neuroactive agent, that provide a sustained release of each active agent. The tablets contain the following ingredients in the amounts indicated:

| **INGREDIENT** | **AMOUNT (mg)** |
|---|---|
| NMDA-RA | 5 - 600 |
| Neuroactive Agent | 1 - 600 |
| Water swellable, erodible and/or soluble polymer that causes a sustained release of the drugs | 10-200 |
| Disintegrant | 3-50 |
| Binder | 3-50 |
| Plasticizer (optional) | 0.7-10 |
| Glidant (optional) | 0.3-5 |
| Lubricant (optional) | 1-15 |
| Diluent (optional) | 2-250 |

The above-described general formulation is used to prepare matrix tablets containing a combination of two different classes of drugs. The first class of drugs is an NMDA-RA, and the second class of drugs is independently selected from the group consisting of an anti-depressant agent and an anxiolytic agent. A first combination includes an NMDA-RA and an anti-depressant. A second combination includes and NMDA-RA and an anxiolytic agent.

In one embodiment, the anti-depressant is selected from the group consisting of an SSRI and an SNRI. In a specific embodiment, the SSRI is selected from the group consisting of fluoxetine, paroxetine, sertraline, fluvoxamine citalopram, and escitalopram. In another specific embodiment, the SNRI is selected from the group consisting of venlafaxine, milnacipran, and duloxetine.

In another embodiment, the anxiolytic agent is selected from the group consisting of benzodiazepines and non-benzodiazepines. In a specific embodiment, the benzodiazepine is selected from the group consisting of lorazepam and oxazepam. In another specific embodiment, the non-benzodiazepine is selected from the group consisting of buspirone and trazodone.

In yet another embodiment, the NMDA-RA is selected from the group consisting of AMN, MEM, and ifenprodil.

Accordingly, the following combinations of dual sustained release dosage forms are prepared according to this example wherein the drugs below are used in matrix tablets.

| Neuroactive agent | NMDA-RA | | | |
|---|---|---|---|---|
| | AMN | MEM | Budipine | ifenprodil |
| Lorazepam | X | | X | X |
| Oxazepam | | X | X | |
| Buspirone | X | X | X | X |
| Trazodone | X | X | X | X |
| Venlafaxine | X | X | X | X |
| Milnacipran | | X | X | |
| Duloxetine | X | | X | X |
| Fluoxetine | X | | X | |
| Paroxetine | | X | X | X |
| Sertraline | | X | X | X |
| Fluvoxamine | X | | X | |
| Citalopram | X | X | X | |
| Escitalopram | | | X | X |
| "X" denotes a drug combination that can be used. | | | | |

It should be understood, the drug combinations set forth above are not limited to use in just a matrix tablet formulation. They can be included in any of the formulations disclosed herein for practice of the method(s) of treatment described herein.

### EXAMPLE 13

### Capsule containing Amantadine (Delayed and CR) and Citalopram (IR)

The following procedure is used to prepare capsules containing Amantadine HCl (150, 300 and 600 mg strength) and Citalopram HBr (5, 10 and 20 mg strength). The initial release of AMN is delayed with respect to initial release of CIT at least 1 hour. The capsules contain the following ingredients in the amounts indicated:

| **INGREDIENT** | **AMOUNT (mg)** | | |
|---|---|---|---|
| **Amantadine Strength⇒** | 150.00 | 300.00 | 600.00 |
| **Citalopram Strength⇒** | 20.00 | 10.00 | 5.00 |

| **CORE** | | | |
|---|---|---|---|
| Amantadine Hydrochoride | 150.00 | 300.00 | 600.00 |
| Lactose Monohydrate | 4.75 | 24.50 | 18.50 |
| Ethylcelullose | 4.50 | 6.20 | 8.00 |
| Polyethylene Glycol 400 | 0.75 | 1.30 | 2.00 |
| Cellulose Acetophthalate | 5.00 | 10.00 | 20.00 |
| Acetone* | 100.00 | 200.00 | 300.00 |
| Citalopram HBr | 24.98 | 12.49 | 6.25 |
| Sugar | 2.00 | 4.00 | 8.00 |
| Talcum | 0.50 | 1.00 | 1.50 |
| Dried Starch | 7.52 | 10.51 | 5.75 |
| Purified water* | 50.00 | 100.00 | 150.00 |
| Capsule Weight | 200.00 | 370.00 | 670.00 |

A first microgranulate (group of microgranules) is prepared as follows. Amantadine HCl and lactose monohydrate are mixed for 5 minutes in a fluid bed. Ethylcellulose, polyethylene glycol 400 are added to acetone to form a solution. This solution is sprayed onto the granules in a fluid bed to obtain coated microgranules. To continuation Cellulose Acetophthalate, polyethylene glycol 400 are added to acetone to form a solution, and this solution is sprayed onto the granules in the fluid bed to obtain enteric coated microgranules.

A second microgranulate is prepared as follows. Citalopram hydrobromide and and dried starch are mixed in a fluid bed for 5 minutes. Sugar and Talcum are added to purified water to form a coating suspension. This suspension is sprayed onto the granules in a fluid bed to obtain coated microgranules.

Both microgranulates are placed into appropriately sized capsule shells. The weight ratio of first to second microgranulates in this example is about 1:1; however, the ratio can be adjusted as needed to provide a capsule dosage form with the appropriate strengths of active agents.

### EXAMPLE 14

### Capsule containing Venlafaxine (CR) and Memantine (Delayed and IR)

The following procedure is used to prepare capsules containing Venlafaxine HCl (37.50, 75.00 and 150.00 mg strength) and Memantine HCl (10, 20 and 40 mg strength). The initial release of MEM is delayed with respect to initial release of VFX at least 1 hour. The capsules contain the following ingredients in the amounts indicated:

| **INGREDIENT** | **AMOUNT (mg)** | | |
|---|---|---|---|
| **Venlafaxine Strength⇒** | 37.50 | 75.00 | 150.00 |
| **Memantine Strengths⇒** | 10.00 | 20.00 | 40.00 |

| **CORE** | | | |
|---|---|---|---|
| Venlafaxine Hydrochoride | 42.43 | 84.86 | 169.72 |
| Lactose Monohydrate | 33.44 | 73.40 | 69.88 |
| Ethylcelullose | 9.00 | 12.40 | 16.00 |
| Polyethylene Glycol 400 | 1.10 | 1.77 | 2..30 |
| Acetone* | 100.00 | 200.00 | 300.00 |
| Memantine Hydrochoride | 12.03 | 24.07 | 36.10 |
| Cellulose Acetophthalate | 2.00 | 3.50 | 6.00 |
| Capsule Weight | 100.00 | 200.00 | 300.00 |

A first microgranulate (group of microgranules) is prepared as follows. Venlafaxine HCl and lactose monohydrate are mixed for 5 minutes in a fluid bed. Ethylcellulose, polyethylene glycol 400 are added to acetone to form a solution. This solution is sprayed onto the granules in a fluid bed to obtain coated microgranules.

A second microgranulate (group of microgranules) is prepared as follows. Memantine HCl and lactose monohydrate are mixed for 5 minutes in a fluid bed. Ethylcellulose, polyethylene glycol 400 are added to acetone to form a solution. This solution is sprayed onto the granules in a fluid bed to obtain coated microgranules. To continuation Cellulose Acetophthalate, polyethylene glycol 400 are added to acetone to form a solution, and this solution is sprayed onto the granules in the fluid bed to obtain enteric coated microgranules.

Both microgranulates are placed into appropriately sized capsule shells. The weight ratio of first to second microgranulates in this example is about 1:1; however, the ratio can be adjusted as needed to provide a capsule dosage form with the appropriate strengths of active agents.

The above is a detailed description of particular embodiments of the invention. It is recognized that departures from the disclosed embodiments may be made within the scope of the invention and that obvious modifications will occur to a person skilled in the art. Those of skill in the art should, in light of the present disclosure, appreciate that many changes can be made in the specific embodiments which are disclosed herein and still obtain a like or similar result without departing from the spirit and scope of the invention. All of the embodiments disclosed and claimed herein can be made and executed without undue experimentation in light of the present disclosure.

## Claims

1. A method of treating or improving the clinical status of a patient suffering from undesired motor movement symptoms associated with one or more disorders by reducing the occurrence, frequency or severity of the one or more motor movement symptoms or by substantially eliminating the occurrence of the one or more motor movement symptoms, the method comprising the steps of:
a. administering an amount of NMDA-RA to a subject suffering from one or more motor movement symptoms associated with one or more disorders; and
b. administering an amount of neuroactive agent to the subject;
c. wherein the drugs are administered for a period of time sufficient to reduce the occurrence, frequency or severity of the one or more motor movement symptoms or to substantially eliminate the occurrence of the one or more motor movement symptoms.

2. The method of claim 1, wherein the motor movement disorder is selected from the group consisting of tremors, dyskinesia, akinesia, rigidity and bradykinesia.

3. The method of claim 1, wherein the one or more disorders is selected from the group consisting of Parkinson's disease, anti-Parkinson's disease drug therapy, and dementia associated with Parkinson's disease.

4. The method of claim 1, wherein the neuroactive agent is selected from the group consisting of an antidepressant and an anxiolytic agent.

5. The method of claim 1, wherein the NMDA-RA is selected from the group consisting of amantadine, memantine, budipine and ifenprodil.

6. The method of claim 4, wherein the neuroactive agent is an antidepressant.

7. The method of claim 6, wherein the antidepressant is selected from the group consisting of a selective serotonin reuptake inhibitor and a serotonin norepinephrine reuptake inhibitor.

8. The method of claim 7, wherein the selective serotonin reuptake inhibitor is selected from the group consisting of fluoxetine, paroxetine, sertraline, fluvoxamine, citalopram, and escitalopram.

9. The method of claim 7, wherein the serotonin norepinephrine reuptake inhibitor is selected from the group consisting of venlafaxine, milnacipran, and duloxetine.

10. The method of claim 5, wherein the NMDA-RA is amantadine.

11. The method of claim 7, wherein the antidepressant is citalopram.

12. The method of claim 4, wherein the neuroactive agent is an anxiolytic agent.

13. The method of claim 12, wherein the anxiolytic agent is of the benzodiazepine class.

14. The method of claim 13, wherein the anxiolytic agent is selected from the group consisting of lorazepam and oxazepam.

15. The method of claim 12, wherein the anxiolytic agent is of the nonbenzodiazepine class.

16. Use of an oral pharmaceutical dosage form comprising a neuroactive agent and an NMDA-RA for the amelioration of motor symptoms associated with one or more disorders.

17. An oral pharmaceutical dosage form comprising effective amounts of amantadine and an antidepressant or an anxiolytic agent.

18. The dosage form of claim 17 comprising amantadine in controlled release form and the antidepressant in controlled or rapid release form.

19. The dosage form of claim 17 comprising amantadine in controlled release form and anxiolytic agent in controlled or rapid release form.

20. The dosage form of claim 18, wherein the dosage form is an osmotic device.

21. The dosage form of claim 19, wherein the dosage form is an osmotic device.

22. An oral dosage form comprising an NMDA-RA in controlled release form and a neuroactive agent in controlled or rapid release form.

23. The dosage form of claim 22, wherein the neuroactive agent is in controlled release form.

24. The dosage form of claim 22, wherein the neuroactive agent is in rapid release form.

25. A method of treating or improving the clinical status of a patient suffering from undesired motor movement symptoms associated with one or more of Parkinson's disease, anti-Parkinson's disease drug therapy, and dementia associated with Parkinson's disease, the method comprising the steps of:
a. orally administering an amount of NMDA-RA to a subject suffering from one or more motor movement symptoms associated with one or more disorders; and
b. orally administering an amount of neuroactive agent to the subject; wherein
c. the NMDA-RA and neuroactive agent are administered orally in a combined amount effective to and for a period of time sufficient to reduce the occurrence, frequency or severity of the one or more motor movement symptoms or to substantially eliminate the occurrence of the one or more motor movement symptoms;
d. the motor movement disorder is selected from the group consisting of tremor, dyskinesia, akinesia, rigidity and bradykinesia; and
e. the neuroactive agent is selected from the group consisting of an antidepressant and an anxiolytic agent.

26. The method of claim 25, wherein the NMDA-RA is selected from the group consisting of amantadine, memantine, budipine and ifenprodil.

27. The method of claim 25, wherein the antidepressant is selected from the group consisting of a selective serotonin reuptake inhibitor and a serotonin norepinephrine reuptake inhibitor.

28. The method of claim 25, wherein the anxiolytic agent is of the benzodiazepine class.

29. The method of claim 25, wherein the anxiolytic agent is of the nonbenzodiazepine class.

30. A method of improving the clinical status of a subject suffering from undesired motor movement symptoms associated with one or more disorders, the method comprising the step of:
a. orally administering to the subject on a once or twice daily basis an oral dosage form comprising a therapeutically effective or sub-therapeutically effective amount of an NMDA-RA, a therapeutically effective or sub-therapeutically effective amount of a neuroactive agent, and one or more pharmaceutically acceptable excipients,
b. wherein, when the dosage form is administered to the subject, the NMDA-RA is released according to a rapid, controlled or sustained release profile, the neuroactive agent is released according to a rapid, controlled or sustained release profile, and release of one or both the NMDA-RA and the neuroactive agent is optionally delayed a lag period,
c. whereby the occurrence, frequency or severity of the one or more motor movement symptoms is reduced or substantially eliminated.

31. Use of a combination of an amount of NMDA-RA and an amount of neuroactive agent for the manufacture of an oral medicament for the treatment of or improvement of the clinical status of a patient suffering from undesired motor movement symptoms associated with one or more disorders.

32. The use of claim 31, wherein the motor movement symptom is selected from the group consisting of tremors, dyskinesia, akinesia, rigidity and bradykinesia.

33. The use of claim 31, wherein the one or more disorders is selected from the group consisting of Parkinson's disease, anti-Parkinson's disease drug therapy, and dementia associated with Parkinson's disease.

34. The use of claim 31, wherein the NMDA-RA is selected from the group consisting of amantadine, memantine, budipine and ifenprodil.

35. The use of claim 31, wherein the neuroactive agent is selected from the group consisting of an antidepressant and an anxiolytic agent.

36. The use of claim 35, wherein the neuroactive agent is an antidepressant.

37. The use of claim 36, wherein the antidepressant is selected from the group consisting of a selective serotonin reuptake inhibitor and a serotonin norepinephrine reuptake inhibitor.

38. The use of claim 37, wherein the selective serotonin reuptake inhibitor is selected from the group consisting of fluoxetine, paroxetine, sertraline, fluvoxamine citalopram, and escitalopram.

39. The use of claim 37, wherein the serotonin norepinephrine reuptake inhibitor is selected from the group consisting of venlafaxine, milnacipran, and duloxetine.

40. The use of claim 37, wherein the NMDA-RA is selected from the group consisting of amantadine, memantine, budipine and ifenprodil.

41. The use of claim 31, wherein the NMDA-RA is amantadine.

42. The use of claim 41, wherein the antidepressant is citalopram.

43. The use of claim 35, wherein the neuroactive agent is an anxiolytic agent.

44. The use of claim 43, wherein the anxiolytic agent is of the benzodiazepine class.

45. The use of claim 44, wherein the anxiolytic agent is selected from the group consisting of lorazepam and oxazepam.

46. The use of claim 43, wherein the anxiolytic agent of the nonbenzodiazepine class.

47. The use of claim 46, wherein the anxiolytic agent is selected from the group consisting of buspirone and trazodone.

48. The method of claim 15, wherein the anxiolytic agent is selected from the group consisting of buspirone and trazodone.
